(19) Europäisches Patentamt
European Patent Office
Office européen des brevets

(11) **EP 4 052 265 B1**

(12) **EUROPEAN PATENT SPECIFICATION**

(45) Date of publication and mention
of the grant of the patent:
**06.05.2026 Bulletin 2026/19**

(21) Application number: **20812425.5**

(22) Date of filing: **28.10.2020**

(51) International Patent Classification (IPC):
***G16H 20/40*** *(2018.01)* ***G16H 40/63*** *(2018.01)*
***G16H 50/20*** *(2018.01)*

(52) Cooperative Patent Classification (CPC):
**G16H 50/20; G16H 20/40; G16H 40/63**

(86) International application number:
**PCT/IB2020/060073**

(87) International publication number:
**WO 2021/084425 (06.05.2021 Gazette 2021/18)**

(54) **VECTORIAL CALCULATION APPLIED TO A DECISION-MAKING MATRIX FOR THE TITRATION OF THE ANAESTHESIA LEVELS**

AUF EINE ENTSCHEIDUNGSMATRIX ZUR TITRATION DER NARKOSESPIEGEL ANGEWANDTE VEKTORIELLE BERECHNUNG

CALCUL VECTORIEL APPLIQUÉ À UNE MATRICE DE PRISE DE DÉCISION DESTINÉ AU TITRAGE DES NIVEAUX D'ANESTHÉSIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2019 IT 201900019888**

(43) Date of publication of application:
**07.09.2022 Bulletin 2022/36**

(73) Proprietor: **Fondazione IRCCS Istituto Nazionale dei Tumori
20133 Milano (MI) (IT)**

(72) Inventor: **TOGNOLI, Emiliano
20133 Milano MI (IT)**

(74) Representative: **Perani & Partners S.p.A.
Corso Europa, 15
20122 Milano (IT)**

(56) References cited:
**US-A1- 2011 105 914      US-A1- 2012 095 437**

**Description**

**FIELD OF THE INVENTION**

The present invention relates to a method for controlling the concentrations of drugs used in anaesthesia. In particular, the method of the present invention implements a series of steps which allow to optimize the modulation of the anaesthesia concentration for the induction and maintenance of a balanced anaesthesia, as well as for the cessation of the anaesthesia in the awakening step.

**STATE OF THE ART**

General anaesthesia mainly includes two components: hypnosis and control of the stress response to surgical trauma; in this sense, anaesthesia can be maintained by the systemic administration of a hypnotic and an analgesic so that the therapeutic objectives of the anaesthesia are achieved by the combined action of the two drugs[1,2]. This anaesthetic technique is generally referred to as "balanced anaesthesia".

Both the hypnotic agent and the analgesic agent contribute, at least in part, to obtaining the two components of the anaesthesia and interact synergistically for the creation of the anaesthetic state[3,4,5]. The synergistic interaction allows the use of lower doses of each drug to achieve a balanced anaesthesia, potentially reducing the occurrence of side effects. Several studies have been designed to identify the optimal concentration of hypnotic and opioid combinations suitable for ensuring an adequate anaesthesia, ensuring the lowest patient exposure to the anaesthetic agents.

One issue which is still unresolved in this clinical context concerns the method to be applied to modulate the level of anaesthesia during the induction, maintenance and awakening steps, whenever the selected target concentrations are inappropriate to control the patient's reaction to procedure-related stress. To control the level of a balanced anaesthesia it is necessary to answer two questions: which drug needs to be adjusted and how the concentration thereof needs to be modulated.

The mathematical model describing the pharmacodynamic interactions between the analgesic agent and the hypnotic agent makes it possible to identify infinite combinations of concentrations which produce the same effect, so that different titration strategies can be put into practice when the patient deviates from an optimal anaesthesia state.

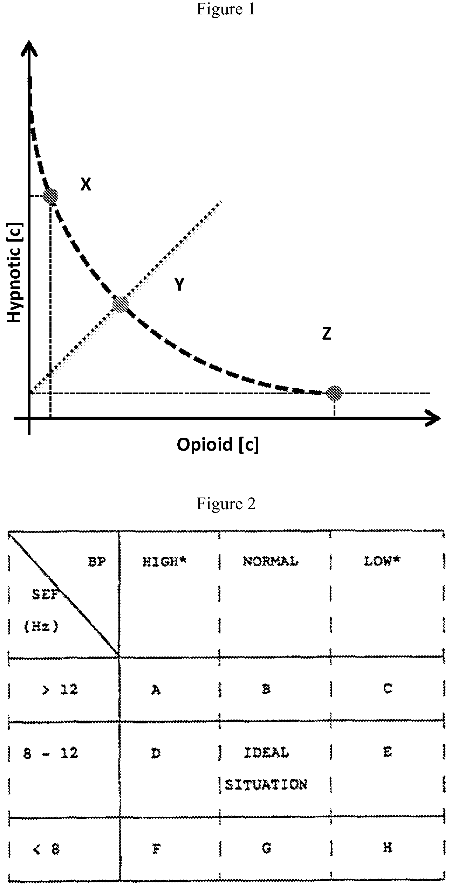

Figure 1 graphically depicts the approaches taken over time by anaesthesiologists, consistent with what has been disclosed in the literature and what has been introduced in clinical practice: studies on the synergy of hypnotics and opioids indicate that a certain level of anaesthesia, measured as a response to increasing stressful stimuli or as a level of intensity of hypnosis, can be obtained by employing (X) high concentrations of hypnotics and low concentrations of opioids, (Y) low concentrations of both, (Z) minimum concentrations of hypnotics and high concentrations of opioids.

By the same token, the separate monitoring of the distinct pharmacodynamic signals of the two drugs cannot solve the dilemma.

An alternative approach consists in combining vital parameters identifying the level of hypnosis or stress response in a matrix, such as that proposed by G.M. Gurman in a publication of 1994[6] (Figure 2). This matrix is based on an electroencephalogram and blood pressure signal as control variables; for both parameters, a range of values can be defined which identifies an optimal anaesthesia region.

In relation to that region, Gurman identifies nine different clinical conditions characterized by the combination of an appropriate increase or decrease of one or both vital parameters, in response to which an increase or decrease of one or both anaesthetic agents is planned.

*Problems of the background art*

The Gurman matrix allows, in view of the patient's vital parameters, to understand on which drug to intervene to balance the anaesthesia, but does not indicate how to intervene on hypnotic and/or analgesic concentrations to obtain the balanced anaesthesia.

State-of-the-art approaches to modulating the hypnotic and analgesic concentration during anaesthesia are based on the experience of the anaesthesiologist and an unstructured approach, which results in an uncoordinated management of the hypnotic and analgesic component of anaesthesia.

US 2012/095437 A1 relates to an automatic control system and method for the control of anaesthesia . Parameters that can be used to monitor the hypnotic component of anaesthesia include the bi-spectral index (BIS), the so-called analgoscore, a score based on the processing of mean arterial pressure (MAP) and heart rate. However, said document only considers these parameters in isolation.

## SUMMARY OF THE INVENTION

The invention is defined by the appended claims.

The Applicant has now found that it is possible to solve the problems of the known art by introducing a vectorial calculation which quantifies the deviation of the anaesthesia induced in the patient with respect to the values expected from a balanced anaesthesia and, through an algorithm, transforms the degree of deviation into a balanced intervention on the hypnotic and analgesic concentrations.

In particular, an object of the present invention is to provide a method for the control of the concentration of the drugs used in anaesthesia so as to facilitate the choice of the parameter to be modified to optimize the steps of inducing and maintaining a balanced anaesthesia, as well as the awakening step.

The mentioned technical task and the specified aims are substantially achieved by a method for the control of hypnotic and anaesthetic concentrations of an anaesthetic composition comprising the technical features set out in one or more of the appended claims.

*Advantages of the Invention*

The inventive method advantageously allows several anaesthesia-related variables to be combined together into a single matrix, in order to pursue the objective of a balanced anaesthesia.

Unlike the Gurman matrix, the method of the invention allows not only to decide on which drug to intervene, but also to intervene quantitatively on the concentration of anaesthetic agents, in order to modulate the adaptation of the anaesthetic plan.

Advantageously, the method is based on a single algorithm capable of simultaneously managing the hypnotic and analgesic components of the anaesthesia.

As mentioned earlier, the modulation of the hypnotic and analgesic concentrations during anaesthesia is currently based on the clinical experience of the anaesthetist, since there is no standardized approach for the treatment of the patient. This is partly due to the fact that the use of combinations of hypnotics and analgesics does not allow the hypnotic effect to be controlled independently of the analgesic effect, since each pharmacological agent (hypnotic and analgesic) independently contributes to both the maintenance of the first and the maintenance of the second pharmacological effect. In fact, it is believed that there is a synergy between hypnotics and analgesics; in practice, this synergy greatly complicates the work of the anaesthetist who proceeds "by trial and error" to develop an anaesthetic plan.

In this sense, the use of a single algorithm to process the clinical indices relating to both the hypnotic state and the analgesic state of the patient, and to return a universal parameter (hereinafter identified as D%) applicable to the increase/decrease calculation of both hypnotic and sedative concentrations, is of great advantage, not only for the simplicity of calculation, but also because it manages to integrate both the hypnotic and analgesic component into the vectorial calculation. This allows the level of anaesthesia to be monitored as a unitary condition, taking into account the two variables which, at the state of the art, would have been analyzed separately while being, from a pharmacological point of view, correlated and influential on each other.

The inventive method aims to standardize the method of administration of anaesthetic drugs, without including any assumptions of the anaesthetist about the mechanism involved in the patient's response to stressful stimuli and anaesthesia.

The method allows to graduate interventions on the patient according to an algorithm which makes patient management uniform; all patients can be managed optimally regardless of the experience or fatigue of the anaesthesiologist.

## DESCRIPTION OF THE FIGURES

Figure 1: Schematic representation of the different pharmacological strategies used to date for the generation of a balanced anaesthesia, as a function of the hypnotic and analgesic concentrations: (X) high hypnotic concentrations and low analgesic concentrations, (Z) low hypnotic concentrations and high analgesic concentrations or (Y) low concentrations of both.

Figure 2: Matrix proposed by Gurman in 1994 which combines the spectral limit frequency (SEF) of the electroencephalogram with blood pressure (BP) to monitor the depth of anaesthesia.

Figure 3: Preferred embodiment of the matrix used for the purpose of the inventive method. The matrix is obtained by the intersection of the dimensions MAP, on the abscissa, and BIS, on the ordinate. MAP varies between 0 and 150 mmHg and BIS varies between 0 and 100. The optimal anaesthesia zone (OAZ) is defined by BIS values between 40 and 60 and MAP values between 65 and 85 mmHg.

Figure 4: Schematic representation of the sub-step of quantifying the deviation of the patient's position (B) with respect to point (A) of the optimal anaesthesia zone (OAZ) by means of vectorial analysis.

Figure 5: Schematic representation of the sub-step of calculating the increase or decrease of the initial hypnotic and/or anaesthetic concentration.

Figure 6: Schematic representation of the clinical case reported in Example 2.

Figure 7: Schematic representation of a preferred embodiment of implementing the method object of the present invention.

Figure 8: Display example of the graphical interface of a computer program configured to implement the steps of the inventive method.

## DETAILED DESCRIPTION OF THE INVENTION

The present invention relates to a method for the control of hypnotic and analgesic concentrations of an anaesthetic composition comprising the steps described below.

The method comprises a step of supplying at least one memory unit configured to receive and store data. Preferably, the memory unit is provided with a plurality of memory areas in which the data received by the data processing unit are stored.

The method comprises a step of supplying at least one data processing unit 101 in signal communication with the memory unit and configured to process and store data in the memory unit.

According to a preferred embodiment, the method comprises a step of providing a graphical interface device 102, in signal communication with the memory unit and the data processing unit 101, and configured to display data stored in the memory unit and/or processed by the data processing unit 101.

According to a preferred embodiment, the method comprises a step of providing a machine for delivering at least one or more drugs 103, said device being in signal communication with the memory unit, with the data processing unit 101, possibly with the graphical interface device 102, and configured to deliver drugs as a function of the signal stored in the memory unit and/or processed by the data processing unit 101.

The method comprises a step of providing the bispectral index ($P_{BIS}$) and mean arterial pressure ($P_{MAP}$) of a patient treated with a first anaesthetic composition comprising an initial hypnotic concentration and an initial analgesic concentration, and storing the patient's $P_{BIS}$ and $P_{MAP}$ data in said memory unit.

Bispectral index (BIS) is intended as a numerical parameter derived from the electroencephalogram of a patient, used to evaluate the level of hypnosis under anaesthesia. The bispectral index commonly ranges from 0 to 100 and correlates with the clinical extremes of anaesthesia. BIS is a hypnosis parameter which gives a measurement of the patient's brain state and not the measurement of the concentration of a particular drug.

The bispectral index is measured according to techniques known to those skilled in the art, such as for example those described by Bruhn J. et al. ("Depth of anaesthesia monitoring: what's available, what's validated and what's next?", J. Bruhn, P. S. Myles, R. Sneyd, M. M. R. F. Struys, BJA: British Journal of Anaesthesia, Volume 97, Issue 1, July 2006, Pages 85-94, https://doi.org/10.1093/bja/ael120).

Mean arterial pressure (MAP) is defined as the mean arterial pressure during a cardiac cycle and is correlated, under anaesthesia, with the patient's response to stressful stimuli, such as surgery or manipulation. The MAP index is influenced by cardiac output and systemic vascular resistance, each of which is under the influence of several variables, known to those skilled in the art. MAP is commonly used in clinical practice to measure the patient's stress response as well as stress due to tissue hypoperfusion.

Blood pressure is the most common hemodynamic parameter recorded during anaesthesia. It can be measured by monitoring systems that detect the data invasively, by positioning a dedicated catheter in a patient's artery (commonly the radial artery), or non-invasively by positioning a sleeve connected to an appropriate monitoring system around the patient's arm. Anaesthesia monitors use the oscillometric method for non-invasive blood pressure measurement. Techniques known to the those skilled in the art for MAP measurement are those described in the literature by Kai Kuck and AI. (Periopera6ve Noninvasive Blood Pressure Monitoring. Anaesth Analg 2018;127:408-11) and by Karsten Bartels and AI. (Blood Pressure Monitoring for the Anaesthesiologist: A Practical Review. Anaesth Analg 2016;122:1866-79).

For the purpose of implementing the method of the invention, the MAP measurement is preferably conducted with a continuous, non-invasive method which measures pressure at the level of the patient's fingers.

The method comprises a step of preparing a two-dimensional matrix defined, on the abscissa, by a MAP dimension relative to the variation of Mean Arterial Pressure, and on the ordinate, by a BIS dimension relative to the variation of the Bispectral Index.

Preferably, the step of arranging a two-dimensional matrix comprises the sub-step of arranging an algorithm, resident in the data processing unit 101 and configured to define the two-dimensional matrix by means of a MAP dimension, on the abscissa, relative to the variation of Mean Arterial Pressure; and by a BIS dimension, on the ordinate, relative to the variation of the Bispectral Index.

The method comprises a step of defining, preferably using the algorithm, the optimal anaesthesia zone (OAZ) located between MAP values of 65 and 110 mmHg and BIS values of 40 and 60.

It should be noted that the range of the variation of the MAP and BIS dimension in the optimal anaesthesia zone (OAZ) is chosen to correlate with important clinical objectives: a MAP value of 55 mmHg is a limit value for distinguishing between moderate or severe hypotension; a MAP value of less than 55 mmHg is clinically associated with the development of postoperative renal and myocardial damage, as well as a worse outcome at 30 days[7].

A MAP value of 110 mmHg is a limit value for distinguishing between a moderate stress reaction and a severe stress reaction.

According to a preferred embodiment, the optimal anaesthesia zone (OAZ) is located between MAP values of 65 and 85 mmHg and BIS values of 40 and 60.

Advantageously, the range of MAP variation within the optimal anaesthesia zone (OAZ), i.e., between 65 and 85 mmHg, allows to keep the patient away from the limit values of 55 mmHg and 110 mmHg.

Without seeking to be constrained by any theory, the Applicant believes that a MAP range between 65 and 85 mmHg may restrict variability in intraoperative stress control.

Regarding the variation in BIS, prospective studies have shown that BIS values maintained between 40 and 60 during anaesthesia ensure an adequate hypnotic state[8]; a BIS value of 30 allows a distinction to be made between moderate or severe deep hypnosis.

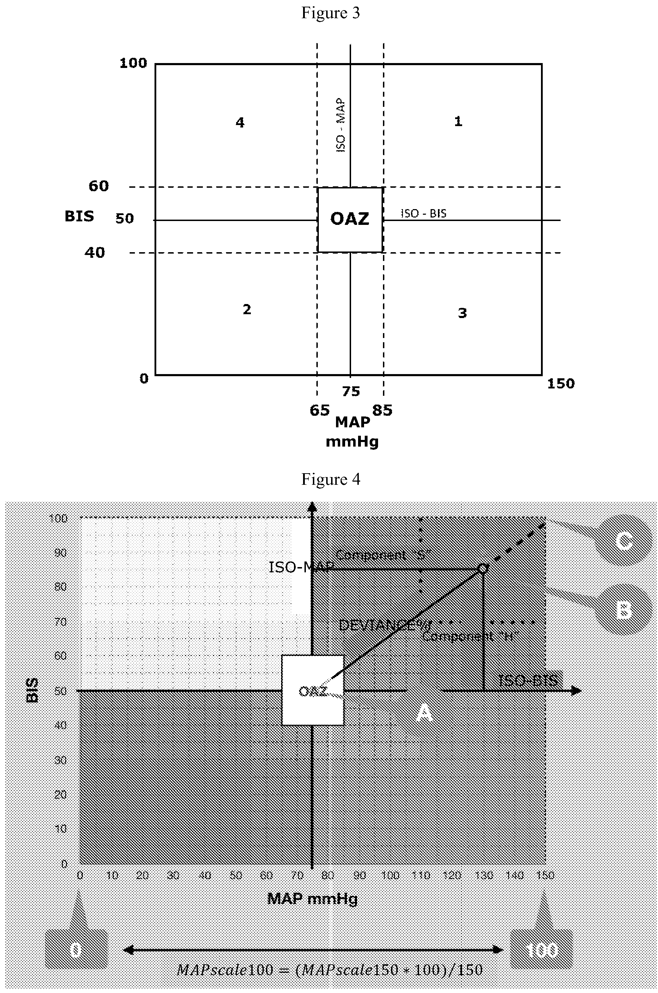

Figure 3 shows, by way of illustration and not limitation, the preferred embodiment of the matrix used in the method of the present invention.

According to a preferred embodiment, the matrix is defined by a MAP variation of 0 to 150 mmHg and a BIS variation of 0 to 100 where the MAP and BIS dimensions orthogonally intersect each other at the zero point (0;0).

The method also comprises a further step of defining in the matrix, preferably via the algorithm, an ISO-MAP axis and an ISO-BIS axis, orthogonal to ISO-MAP, the intersection of which defines a point (A) of the optimal anaesthesia zone (OAZ).

According to a preferred embodiment, the point (A) is defined by the intersection of the ISO-MAP axis, at a MAP value of 75 mmHg, and the ISO-BIS axis, at a BIS value of 50.

The method comprises a step of localizing in the matrix, via the algorithm, the values of the $P_{BIS}$ and $P_{MAP}$ data, to define a patient's position (B) in the matrix with respect to the optimal anaesthesia zone (OAZ).

The method comprises a step of providing an algorithm resident in said processing unit, configured to quantify the deviation of the patient's position (B) with respect to the optimal anaesthesia zone (OAZ) and transforming the deviation into a quantitative variation of the initial hypnotic concentration and/or initial analgesic concentration of the first anaesthetic composition to define a target hypnotic concentration and/or a target analgesic concentration of a second anaesthetic composition.

According to a preferred embodiment, the algorithm configured to quantify the deviation of the patient's position (B) with respect to the optimal anaesthesia zone (OAZ) and transforming the deviation into a quantitative variation of the initial hypnotic concentration and/or of the initial analgesic concentration, is also configured to define the two-dimensional matrix by means of a MAP dimension, on the abscissa, relative to the variation of the Mean Arterial Pressure; and by a BIS dimension, on the ordinate, relative to the variation of the Bispectral Index.

The method comprises a step of processing the patient's $P_{BIS}$ and $P_{MAP}$ data by means of said algorithm to obtain the target hypnotic concentration and/or target anaesthetic concentration of the second anaesthetic composition.

It should be noted that the two ISO-BIS and ISO-MAP axes divide the matrix into four areas, each corresponding to four main clinical situations which the algorithm must interpret (Figure 3): the upper right area (1) is representative of insufficient anaesthesia and is characterized by a concomitant increase in BIS and MAP values; the lower left area (2) is representative of deep anaesthesia and is characterized by decreasing BIS and MAP values. The lower right area (3) is representative of inappropriate anaesthesia, both in terms of hypnosis and in terms of analgesia; this area is characterized by decreasing BIS values and increasing MAP values. The upper left area (4) is representative of an insufficient anaesthesia condition and is characterized by an increase in BIS values and a decrease in MAP values.

The areas (3) and (4) are the most problematic in that the variations in BIS and MAP follow opposite trends and a different balance of hypnotic and analgesic concentrations must be identified to bring the patient back to the optimal anaesthesia zone (OAZ).

It should be noted that the method of the invention is conditioned by the relative width of the dimensions BIS and MAP; in this sense, a greater percentage deviation is attributable to the dimension characterized by a wider variation.

In order to make the BIS range and the MAP range equivalent, the method of the invention preferably comprises a further step of transforming the range of variation of the MAP dimension from a scale 0-150 (MAPscala150) to a scale 0-100 (MAPscala100).

Preferably, the 0-100 scale MAP transformation (MAPscala100) is calculated with the expression below:

$$Formula\ 2 \qquad MAPscale100 = (MAPscale150 * 100)/150$$

According to a preferred embodiment, the step of processing the patient's $P_{BIS}$ and $P_{MAP}$ data by means of the algorithm comprises the sub-steps of:

- quantifying the deviation of the patient's position (B) with respect to point (A) of the optimal anaesthesia zone (OAZ) by means of a vectorial calculation,
- decomposing the deviation of the patient's position (B) with respect to point (A) of the optimal anaesthesia zone (OAZ) into a deviation from hypnosis (H) and/or a deviation from analgesia (S) with respect to point (A) of the optimal anaesthesia zone (OAZ), and
- calculating an increase or decrease in the initial hypnotic and/or analgesic concentration of the first anaesthetic composition as a function of said deviations ($D_H$; $D_S$).

Preferably, the sub-step of quantifying the deviation of the patient's position (B) with respect to point (A) of the optimal anaesthesia zone (OAZ) by means of a vectorial calculation comprises a sub-step of defining a vector (V) passing through point (A) of the optimal anaesthesia zone (OAZ), through the patient's position (B).

Figure 4 graphically represents the procedure by which the algorithm performs the sub-step of quantifying the deviation of the patient's position (B) with respect to point (A) of the optimal anaesthesia zone (OAZ) by means of a vectorial calculation.

Preferably, the sub-step of quantifying the deviation of the patient's position (B) with respect to point (A) of the optimal anaesthesia zone (OAZ) by means of a vectorial calculation comprises a sub-step of calculating a percentage deviation (D%) of the patient's position (B) with respect to point (A) of the optimal anaesthesia zone (OAZ), on the maximum deviation (C) of the MAP and BIS values along the vector (V).

In more detail, the percentage deviation (D%) is obtained with the following expression:

$$Formula\ 3 \qquad D\% = \frac{A-B}{A-C} * 100$$

where:

- A-B corresponds to the distance between the patient's position (B) and point (A) of the optimal anaesthesia zone;
- A-C corresponds to the distance between point (A) of the optimal anaesthesia zone (OAZ) and the maximum deviation (C) of MAP and BIS along the vector (V).

According to a preferred embodiment, the distance (A-B) between the patient's position (B) and point (A) of the optimal anaesthesia zone is calculated with the Pythagorean Theorem (Figure 4).

Preferably, the distance (A-B) between the patient's position (B) and point (A) of the optimal anaesthesia zone is obtained with the following expression:

$$Formula\ 4 \qquad A\text{-}B = \sqrt{(AE^2 + BE^2)}$$

where:

- A-E corresponds to the distance between point (A) of the optimal anaesthesia zone and the projection on the ISO-BIS axis (E) of the patient's position (B).

- B-E corresponds to the distance between the patient's position (B) and the projection on the ISO-BIS axis (E) of the patient's position (B).

According to a preferred embodiment, the distance (A-C) between point (A) of the optimal anaesthesia zone (OAZ) and the maximum deviation (C) of MAP and BIS along the vector (V) is calculated with the Pythagorean Theorem.

Preferably, the distance (A-C) between point (A) of the optimal anaesthesia zone (OAZ) and the maximum deviation (C) of MAP and BIS along the vector (V) is obtained with the following expression:

$$Formula\ 5 \qquad A\text{-}C = \sqrt{(AD^2 + CD^2)}$$

where:

- A-D corresponds to the distance between point (A) of the optimal anaesthesia zone and the projection on the ISO-BIS axis (D) of the maximum deviation (C) of MAP and BIS along the vector (V).

- C-D corresponds to the distance between the maximum deviation (C) of MAP and BIS along the vector (V) and the projection on the ISO-BIS axis (D) of the maximum deviation (C) of MAP and BIS along the vector (V).

This maximum deviation (C) of the MAP and BIS values along the vector (V) corresponds to the point of intersection between the vector (V), passing through point (A) and the patient's position (B), and the perimeter of the matrix.

The definition of the vector (V) allows to characterize the deviation of the parameters $P_{BIS}$ and $P_{MAP}$ from point (A) of the optimal anaesthesia zone.

It should be noted that the matrix has an angular profile, and for this reason, the distance between the maximum deviation (C) of the MAP and BIS values and point (A) is not constant. The quantification of the deviation of the patient's position (B) with respect to the optimal anaesthesia zone (OAZ) in terms of percentage deviation (D%) advantageously allows to normalize the data, weighing, in each case, the deviation of the patient's position (B) with respect to the maximum deviation (C) of MAP and BIS.

Preferably, the sub-step of decomposing the deviation of the patient's position (B) with respect to the optimal anaesthesia zone (OAZ) comprises a sub-step of calculating the deviation from hypnosis ($D_H$) by applying the percentage deviation (D%) of the patient's position (B) with respect to point (A) of the optimal anaesthesia zone (OAZ) to a hypnotic coefficient (H').

In more detail, the deviation from hypnosis ($D_H$) is obtained by the following expression:

$$Formula\ 6 \qquad D_H = H' * D\%$$

and the hypnotic coefficient (H') is obtained with the following expression:

$$Formula\ 7 \qquad H' = \frac{H}{|H|+|S|}$$

where

- H' is the hypnotic coefficient, obtained by dividing a hypnotic vector component (H) by the sum of the hypnotic vector component (H) and an analgesic vector component (S);

- |H| is the hypnotic vector component and corresponds to the distance between the patient's position (B) and the ISO-BIS axis (B-E), in absolute value;

- S is the analgesic vector component and corresponds to the distance between the patient's position (B) from the ISO-MAP axis, in absolute value. Note that the vector component S equals, in dimensional terms, the distance A-E between point (A) of the optimal anaesthesia zone and the projection on the ISO-BIS axis (E) of the patient's position (B) (Figure 4).

In more detail, the hypnotic vector component H is obtained with the following expression:

$$Formula\ 8 \qquad H = P_{BIS} - ISO\text{-}BIS$$

According to a preferred embodiment, when the ISO-BIS axis is defined by a BIS value of 50, the hypnotic vector component H is obtained with the following expression:

$$\textit{Formula 9} \qquad\qquad H = P_{BIS} - 50$$

Similarly, the analgesic vector component S is obtained with the following expression:

$$\textit{Formula 10} \qquad\qquad S = \frac{(P_{MAP} - ISO\text{-}MAP)}{D(MAP)} * 100$$

where
D(MAP) is the MAP dimension value of the projection on the ISO-BIS axis (D) of the maximum deviation (C) of MAP and BIS along the vector (V).

According to a preferred embodiment, when the ISO-MAP axis is defined by a MAP value of 75, the analgesic vector component S is obtained with the following expression:

$$\textit{Formula 11} \qquad\qquad S = \frac{(P_{MAP} - 75)}{D(MAP)} * 100$$

According to a preferred embodiment, when the matrix is defined by a MAP variation between 0 and 150 mmHg, D(MAP) is 150 mmHg and the analgesic vector component S is obtained with the following expression:

$$\textit{Formula 12} \qquad\qquad S = \frac{(P_{MAP} - 75)}{150} * 100$$

Similarly, the sub-step of decomposing the deviation of the patient's position (B) with respect to point (A) of the optimal anaesthesia zone (OAZ) comprises the sub-step of calculating the deviation from analgesia ($D_S$) by applying the percentage deviation (D%) of the patient's position (B) with respect to point (A) of the optimal anaesthesia zone (OAZ) to an analgesia coefficient (S').

In more detail, the deviation from analgesia ($D_S$) is obtained by the following expression:

$$\textit{Formula 13} \qquad\qquad D_S = S' * D\%$$

and the analgesia coefficient (S') is obtained with the following expression:

$$\textit{Formula 14} \qquad\qquad S' = \frac{S}{|H| + |S|}$$

where the analgesia coefficient (S') is obtained by dividing the analgesic vector component (S) by the sum of the analgesic vector component (S) and the hypnotic vector component (H), where the analgesic vector component (S) and the hypnotic vector component (H) are in absolute value.

It should be noted that the distance from the ISO-BIS axis allows to determine the degree of deviation from the patient's hypnotic state, while the distance from ISO-MAP allows to determine the degree of deviation from the patient's analgesia state, i.e., from a stress-free state.

Preferably, the deviation from hypnosis ($D_H$) and the deviation from analgesia ($D_S$) are used in the sub-step of calculating an increase or decrease in the initial hypnotic and/or analgesic concentration of the first anaesthetic composition.

According to a preferred embodiment, the method comprises a step of providing the minimum hypnotic and analgesic concentration ($[c_{min}]$) and storing such minimum concentration in the memory unit.

It should be noted that minimum concentration is intended as the minimum concentration which can be administered to the patient to produce the desired anaesthetic effect.

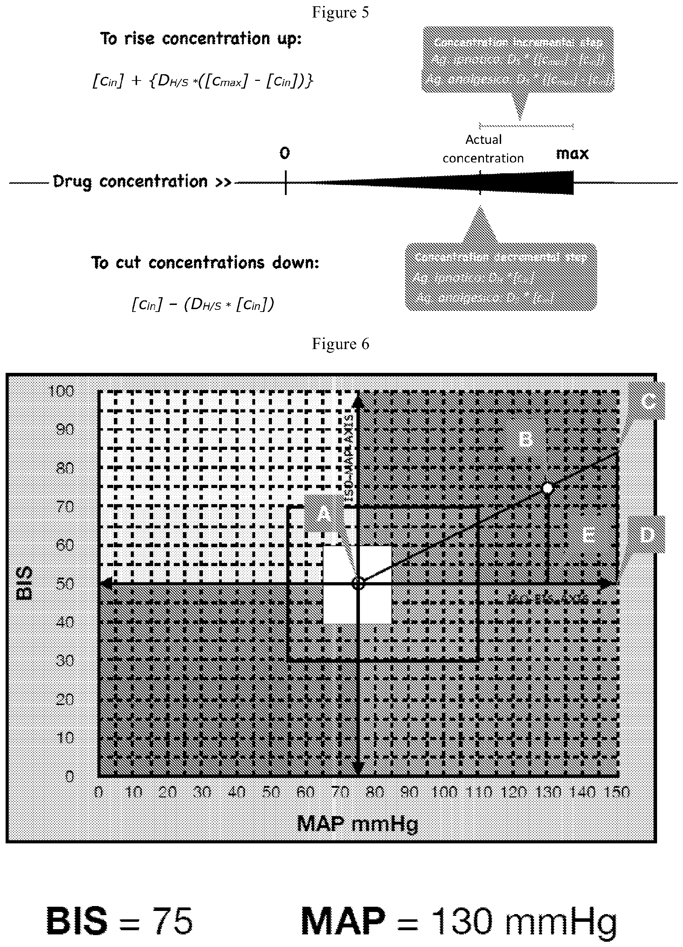

Figure 5 graphically represents the procedure by which the algorithm performs the sub-step of calculating an increase or decrease in the initial hypnotic and/or anaesthetic concentration.

According to an alternative embodiment, the sub-step of calculating an increase or decrease in the initial hypnotic and/or analgesic concentration comprises the sub-step of:

- modifying the hypnotic and/or analgesic concentration if the percentage deviation (D%) of the patient's position (B) from point (A) of the optimal anaesthesia zone (OAZ) is greater than 10%.

Advantageously, the algorithm provides that, within this limit value of percentage deviation (D%), it is not necessary to intervene on the hypnotic and/or analgesic concentrations to be administered to the patient since the deviation from the optimal anaesthesia zone (OAZ) is such that it is negligible.

According to a preferred embodiment, the sub-step of calculating an increase or decrease of the initial hypnotic and/or analgesic concentration comprises the sub-step of

- modifying the hypnotic concentration if the deviation from hypnosis ($D_H$) is greater than 7.5%; and

- modifying the analgesic concentration if the deviation from analgesia ($D_S$) is greater than 7.5%.

Advantageously, the algorithm provides that, within these limit values of deviation from hypnosis ($D_H$) and deviation from analgesia ($D_S$), it is not necessary to intervene on the hypnotic and/or analgesic concentrations to be administered to the patient since the variables under analysis ($D_H$; $D_S$) are outside the optimal anaesthesia zone (OAZ) by an acceptable range.

Preferably, the sub-step of calculating the increase or decrease of the initial hypnotic and/or analgesic concentration allows to transform the deviation from hypnosis ($D_H$) and/or the deviation from analgesia ($D_S$) into a variation delta of the initial hypnotic and/or analgesic concentration.

Preferably, the sub-step of calculating an increase or decrease in the initial hypnotic and/or analgesic concentration comprises the sub-steps of:

- modifying the hypnotic concentration if the percentage deviation (D%) of the patient's position (B) from point (A) of the optimal anaesthesia zone (OAZ) is greater than 10% and, at the same time, the deviation from hypnosis ($D_H$) is greater than 7.5%;

- modifying the analgesic concentration if the percentage deviation (D%) of the patient's position (B) from point (A) of the optimal anaesthesia zone (OAZ) is greater than 10% and, at the same time, the deviation from analgesia ($D_S$) is greater than 7.5%.

It should be noted that, preferably, during the sub-step of calculating an increase or decrease in the initial hypnotic and/or analgesic concentration, the algorithm determines sequentially

1) if the percentage deviation (D%) of the patient's position (B) from point (A) of the optimal anaesthesia zone (OAZ) is greater than or less than 10%;

2) when this percentage deviation (D%) is greater than 10%, the algorithm determines whether the deviation from hypnosis ($D_H$) or from analgesia ($D_S$) is greater than or less than 7.5%

3) when the deviation from hypnosis ($D_H$) or analgesia ($D_S$) is greater than 7.5%, the algorithm calculates an increase or decrease in the initial hypnotic and/or analgesic concentration.

Preferably, the sub-step of calculating a concentration <u>increase</u> comprises the sub-step of:

- applying the deviation from hypnosis ($D_H$) or from analgesia ($D_S$) to the difference between a maximum hypnotic and/or analgesic concentration ($[c_{max}]$) and the initial hypnotic and/or analgesic concentration ($[c_{in}]$), adding it to the initial hypnotic and/or analgesic concentration ($[c_{in}]$), to obtain an increase delta of the initial hypnotic and/or analgesic concentration.

In more detail, the concentration increase is calculated with the expression below:

$$\textit{Formula 15} \qquad [c_{target}]_f = [c_{in}] + \{D_{H/S} * ([c_{max}] - [c_{in}])\}$$

It should be noted that maximum hypnotic and/or analgesic concentration is intended as the maximum concentration which can be administered to the patient, which will produce the desired effect without unacceptable side effects.

For Remifentanil and Propofol, it was possible to determine a simplified formula for calculating a concentration increase; in these cases, the sub-step of calculating a concentration <u>increase</u> preferably comprises the sub-step of:

- applying the deviation from hypnosis ($D_H$) or from analgesia ($D_S$) to an empirical constant (k), adding it to the initial hypnotic and/or analgesic concentration ($[c_{in}]$), to obtain an increase delta of the initial hypnotic and/or analgesic concentration,

where the empirical constant (k) is

3 µg/mL for Propofol; and

6 ng/mL for Remifentanil.

In more detail, the concentration increase for Propofol and Remifentanil is calculated with the following expression:

$$\textit{Formula 15a} \qquad [c_{target}]_\uparrow = [c_{in}] + \{D_{H/S} * k_{Propofol/Remifentanil}\}$$

According to a preferred embodiment, when the hypnotic is Propofol, the concentration <u>increase</u> is calculated with the following expression:

$$\textit{Formula 16} \qquad [c_{\text{target}}]_{\uparrow Propofol} = [c_{in}]_{Propofol} + (D_H * 3)$$

According to a preferred embodiment, when the sedative is Remifentanil, the concentration <u>increase</u> is calculated with the following expression:

$$\textit{Formula 17} \qquad [c_{\text{target}}]_{\uparrow Remifentanil} = [c_{in}]_{Remifentanil} + (D_S * 6)$$

Preferably, the sub-step of calculating a concentration <u>decrease</u> comprises the sub-step of:

- applying the deviation from hypnosis ($D_H$) or from analgesia ($D_S$) to the initial hypnotic and/or analgesic concentration ($[c_{in}]$), subtracting it from the initial hypnotic and/or analgesic concentration ($[c_{in}]$), to obtain a <u>decrease</u> delta of the initial hypnotic and/or analgesic concentration.

In more detail, the concentration decrease is calculated with the following expression:

$$\textit{Formula 18} \qquad [c_{target}]_\downarrow = [c_{in}] - (D_{H/S} * [c_{in}])$$

According to a preferred embodiment, when the hypnotic is Propofol, the concentration <u>decrease</u> is calculated with the following expression:

$$\textit{Formula 19} \qquad [c_{\text{target}}]_{\downarrow Propofol} = [c_{in}]_{Propofol} - ([c_{in}]_{Propofol} * [D_H])$$

According to a preferred embodiment, when the sedative is Remifentanil, the concentration <u>decrease</u> is calculated with the following expression:

$$\textit{Formula 20} \qquad [c_{\text{target}}]_{\downarrow Remifentanil} = [c_{in}]_{Remifentanil} - ([c_{in}]_{Remifentanil} * [D_S])$$

Note that formulas 18, 19, and 20 for calculating the concentration decrease can intervene more drastically on the level of anaesthesia than formulas 15, 15a, 16, and 17 for calculating the concentration increase. This different mathematical approach is mainly justified by the fact that the state of hypotension, often associated with a low BIS value, requires rapid intervention in order to prevent serious organ damage to the heart, brain and kidneys. The limitation of this formula is that the algorithm allows to reset the anaesthetic concentrations and this intervention is not always justified. In the clinical implementation of the invention it will therefore be necessary to provide for a lower concentration limit for each anaesthetic with the function of limiting the intervention of the algorithm. The concentration limits cannot be derived from the vectorial analysis. The lower concentration limits may vary depending on the timing of the procedure, the administration of muscle relaxants or the administration of drugs which contribute to the anaesthetic state, such as Morphine. The lower concentration limits for each step of the surgical procedure should be set based on data from the literature and principles of good clinical practice.

According to a preferred embodiment, when the initial hypnotic or analgesic concentration is modified during the sub-step of calculating an increase or decrease of the initial hypnotic and analgesic concentration, the method comprises the step of

- generating, by means of the algorithm, a datum relative to the target hypnotic and analgesic concentrations of the second anaesthetic composition, this datum being configured to induce the processing unit to generate a receivable signal:

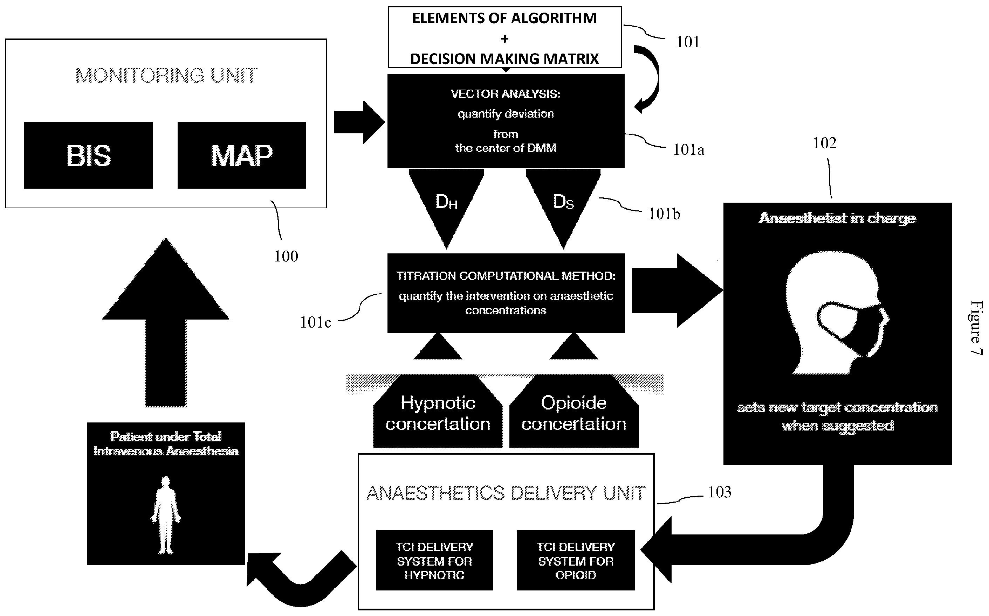

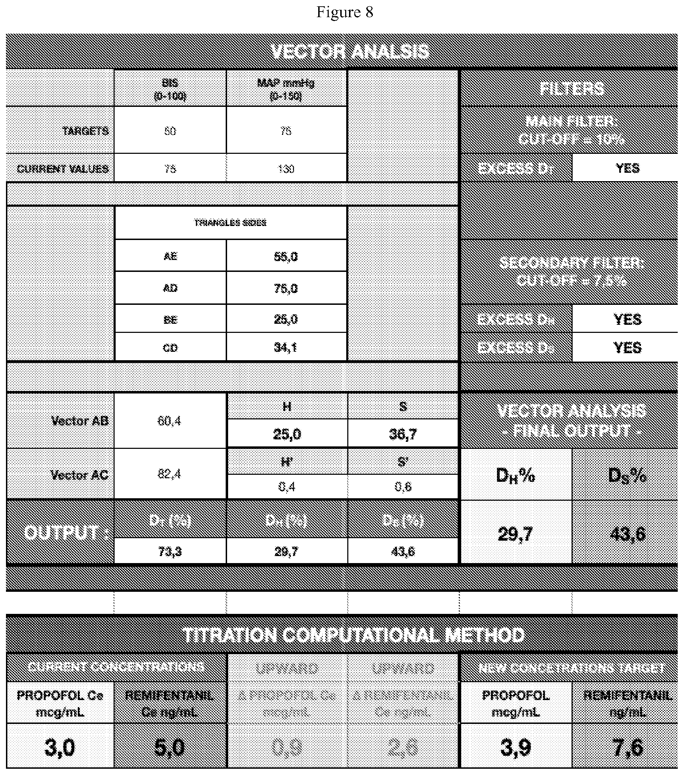

  - from a graphical interface device 102 configured to display target hypnotic and sedative concentrations processed by the algorithm (Figure 8), and/or
  - from a machine configured for the delivery of one or more drugs 103 (Figure 7).

Preferably, the datum relative to the target hypnotic and analgesic concentrations of the second anaesthetic composition is recorded by the processing unit in the memory unit.

Preferably, when the algorithm generates a datum relative to the target hypnotic and analgesic concentrations of the second anaesthetic composition, the method comprises the step of

- generating, by means of the processing unit, a signal as a function of the datum relative to the target hypnotic and analgesic concentrations and sending the signal to a graphical interface device 102 configured to display the target hypnotic and sedative concentrations processed by the algorithm; and/or

- generating a signal as a function of the datum relative to the target hypnotic and analgesic concentrations and sending the signal to a machine configured to deliver one or more drugs 103 as a function of the target hypnotic and sedative concentrations processed by the algorithm.

Preferably, the method of the invention comprises a further step of the anaesthesiologist approving the datum relative to the target hypnotic and analgesic concentration of the second anaesthetic composition by means of the graphical interface.

In particular, when the datum is displayed on the graphical interface, the approval step preferably comprises the sub-step of

- confirming sending the signal relative to the datum of the target hypnotic and analgesic concentrations of the second anaesthetic composition to the delivery machine 103.

In particular, when the datum is sent to the delivery machine 103, or simultaneously to the delivery machine 103 and to the graphical interface device 102, the approval step preferably comprises the sub-step of

- starting the delivery of the drugs, by means of the delivery machine 103, according to the datum of the target hypnotic and analgesic concentration of the second anaesthetic composition.

A further object of the present invention is a system for performing the method of the invention. The system comprises

- a memory unit configured to receive and store data;

- a data processing unit 101 in signal communication with the memory unit and configured to process and store data in the memory unit;

- an algorithm, resident in the data processing unit 101 and configured to

  define a two-dimensional matrix by means of a MAP dimension, on the abscissa, relative to the variation in Mean Arterial Pressure; and by a BIS dimension, on the ordinate, relative to the variation in the Bispectral Index;

  define in the matrix an optimal anaesthesia zone (OAZ) located between MAP values of 65 and 110 mmHg and BIS values of 40 and 60;

  define in the matrix an ISO-MAP axis and an ISO-BIS axis, orthogonal to ISO-MAP, the intersection of which defines a point (A) of the optimal anaesthesia zone (OAZ);

localize the values of the $P_{BIS}$ and $P_{MAP}$ data in the matrix, to define a position (B) of the patient in the matrix with respect to the optimal anaesthesia zone (OAZ);

- an algorithm, resident in the processing unit 101, configured to quantify the deviation of the patient's position (B) with respect to the optimal anaesthesia zone (OAZ) and transforming the deviation into a quantitative variation of the initial hypnotic concentration and initial analgesic concentration of the first anaesthetic composition to define a target hypnotic concentration and a target analgesic concentration of a second anaesthetic composition.

Preferably, the memory unit is provided with a plurality of memory areas in which the data received by the data processing unit is stored.

More preferably, the processing unit is configured to:

- perform the algorithm resident in said processing unit, configured to quantify the deviation of the patient's position (B) with respect to the optimal anaesthesia zone (OAZ) and transforming the deviation into a quantitative variation of the initial hypnotic concentration and initial analgesic concentration of the first anaesthetic composition to define a target hypnotic concentration and a target analgesic concentration of a second anaesthetic composition; and

- process the patient's $P_{BIS}$ and $P_{MAP}$ data by means of said algorithm to obtain a target hypnotic concentration and/or a target anaesthetic concentration of a second anaesthetic composition.

Preferably, the processing unit is configured to perform the algorithm, resident in the processing unit 101 and configured to define the two-dimensional matrix by means of a MAP dimension, on the abscissa, relative to the variation of Mean Arterial Pressure; and by a BIS dimension, on the ordinate, relative to the variation of the Bispectral Index.

According to a preferred embodiment, the system comprises

- a graphical interface device 102, in signal communication with the memory unit and the data processing unit 101, and configured to display data stored in the memory unit and/or processed by the data processing unit 101, and/or

- a machine for delivering one or more drugs 103, said machine being in signal communication with the memory unit, with the data processing unit, possibly with the graphical interface device 102, and configured to deliver drugs as a function of the signal stored in the memory unit and/or processed by the data processing unit 101.

Preferably, the graphical interface device 102 is configured to receive the datum relative to the target hypnotic and analgesic concentration of the second anaesthetic composition; preferably, the datum received is displayed on the device in a graphical interface, by means of a computer program configured for this purpose.

The graphical interface device may be configured to receive and display in the same graphical interface other data resulting from processing the $P_{BIS}$ and $P_{MAP}$ data by means of the algorithm resident in the data processing unit 101; by way of illustration and not limitation, such additional data may be:

- the $P_{BIS}$ and $P_{MAP}$ data recorded in the memory unit by a patient monitoring unit 100; and/or

- data relative to the percentage deviation value (D%) of the patient's position (B) from point (A), the deviation from hypnosis ($D_H$) and deviation from analgesia ($D_S$), and/or

- the two-dimensional matrix and optimal anaesthesia zone (OAZ).

Preferably, the drug delivery machine 103 is configured to deliver at least one hypnotic and one analgesic.

The machine 103 is preferably configured to receive the datum relative to the target hypnotic and analgesic concentration of the second anaesthetic composition and to deliver, based on this datum and preferably upon the approval of the anaesthesiologist, the target hypnotic and analgesic concentration of the second anaesthetic composition, processed by the data processing unit 101 by means of the algorithm.

According to a preferred embodiment, the machine 103 is also configured to detect the plasma concentrations of one or more drugs administered to the patient and generate a datum relative to such information. Preferably, said plasma concentrations of one or more drugs administered to the patient are the initial hypnotic and analgesic concentrations of the first anaesthetic composition. The machine is preferably configured to generate a datum relative to the plasma concentrations of one or more drugs administered to the patient in the memory unit, the datum being configured to induce the data processing unit 101 to record the data from the memory unit and/or to induce the data processing unit 101 to generate a signal receivable by the graphical interface device 102 configured to display the hypnotic and sedative plasma concentrations.

Preferably, the data processing unit 101 is configured to read the datum relative to the initial hypnotic and analgesic concentrations of the first anaesthetic composition, when recorded in the memory unit, and integrate this datum in the processing of the target hypnotic and analgesic concentrations of the second anaesthetic composition by means of the algorithm.

According to a preferred embodiment, the system also comprises a patient monitoring unit 100, provided with sensors configured for measuring the bispectral index ($P_{BIS}$) and mean arterial pressure ($P_{MAP}$) of a patient. This monitoring unit 100 is preferably in signal communication with the memory unit, with the data processing unit, possibly with the graphical interface device 102, possibly with a machine configured for delivering one or more drugs 103.

The patient monitoring unit 100 is preferably configured to generate a datum relative to the bispectral index ($P_{BIS}$) and the mean arterial pressure ($P_{MAP}$) of a patient, the data being configured to induce the data processing unit 101 to record the data in the memory unit.

Preferably, the data processing unit 101 is configured to read the datum relative to the bispectral index ($P_{BIS}$) and mean arterial pressure ($P_{MAP}$), when recorded in the memory unit, and integrate this datum into the processing of the target hypnotic and analgesic concentrations of the second anaesthetic composition by means of the algorithm resident therein.

Figure 7 schematically represents a preferred embodiment of the inventive method, in which said step of approving the datum relative to the target hypnotic and analgesic (sedative) concentrations is contemplated; the diagram allows to understand the technical role which the algorithm covers in the process of controlling the concentrations of the anaesthetic composition. In this case, the method is implemented in a system comprising

- a patient monitoring unit 100, provided with sensors configured for measuring a patient's bispectral index ($P_{BIS}$) and mean arterial pressure ($P_{MAP}$);

- a data processing unit 101 in signal communication with the monitoring unit 100, configured to process the data coming from the monitoring unit 100 by means of the algorithm resident therein, the algorithm being configured for both the predisposition of the two-dimensional matrix and for the determination of the target hypnotic and analgesic concentrations;

- a memory unit (not visible in the figure) in signal communication with the data processing unit 101 and the patient monitoring unit 100, configured to receive and record data from said units;

- a graphical interface device 102, on which the data relative to the target hypnotic and analgesic concentration processed by the data processing unit 101 can be displayed, by means of the algorithm, as well as the patient monitoring data, recorded in the memory unit by the monitoring unit 100; the graphical interface device 102 is the device which allows the anaesthesiologist to view the system output, confirming the sending of the signal relative to the datum of the target hypnotic and analgesic concentrations of the second anaesthetic composition to a delivery machine 103;

- a machine configured for delivering one or more drugs 103 by means of intravenous infusion; the machine 103 is configured to send and receive data from the memory unit and the data processing unit 101; in the specific embodiment depicted in Figure 8, the machine 103 is configured to monitor the plasma concentrations of hypnotic and analgesic infused to the patient and to generate a datum relative to such concentrations, such as to induce the data processing unit 101 to record the data in the memory unit. The data processing unit 101 integrates the datum relative to the (initial) hypnotic and analgesic plasma concentrations in the processing of the increase or decrease in hypnotic and analgesic concentrations by means of the algorithm.

Systems suitable for this purpose are, for example, infusion systems (TCI: target controlled infusion) which convert a certain target concentration, set by the anaesthesiologist, into the corresponding quantity of drug to be delivered through a pump/syringe, in the unit of time, to reach the preset target. This conversion is performed automatically by a microprocessor, contained in the infusion system, programmed with an algorithm which includes the pharmacokinetic model of the drug administered.

With reference to the embodiment depicted in Figure 7, it should be noted that the processing unit 101 provides, by means of the algorithm, a two-dimensional matrix defined by the MAP (0-150 mmHg) and BIS dimensions (0-100); the processing unit detects the patient's $P_{BIS}$ and $P_{MAP}$ data recorded by the monitoring unit 100 in the memory unit and processes them, by means of the algorithm, with a vector analysis (example 2) 101a: the vector analysis serves to quantify the percentage deviation (D%) of the patient's position (B) from the optimal anaesthesia zone (OAZ); if the algorithm determines that the percentage deviation (D%) is greater than 10%, the algorithm proceeds to the analysis of the hypnotic ($D_H$) and analgesic ($D_S$) vector components 101b; if the algorithm determines that at least one of the hypnotic ($D_H$) and analgesic ($D_S$) vector components is greater than 7.5%, the algorithm proceeds to calculate the increase or decrease to be applied to the initial hypnotic and analgesic concentrations 101c. The algorithm then generates a datum relative to the increase or decrease of the initial hypnotic and analgesic concentrations and induces the processing unit to generate a signal configured to be displayed on a graphical interface device 102. The anaesthesiologist, viewing the data on the graphical interface, proceeds or not to the approval step, confirming, where deemed appropriate, the sending of the signal relative to the datum of the target hypnotic and analgesic concentrations of the second anaesthetic composition to the delivery machine 103.

An example of how the datum relative to the increase or decrease of the initial hypnotic and analgesic concentrations is displayable on a graphical interface of a computer program, configured to implement the method of the invention, is represented in Figure 8. The graphical interface displays the $P_{BIS}$ and $P_{MAP}$ values (current values) as well as the target $P_{BIS}$ and $P_{MAP}$ values intended to be achieved (targets) for the induction, maintenance and termination (awakening) of a controlled anaesthesia. In the embodiment of the graphical interface of Figure 8, the values of the segments A-E, A-D, B-E, C-D, defined on the two-dimensional matrix for the determination of the distance A-B of the patient's position (B) from the optimal anaesthesia zone (OAZ), by the Pythagorean theorem, are displayed. The graphical interface displays the output of the calculations deriving from the vector analysis and specifically, the hypnotic coefficient (H') and the analgesia coefficient (D'), the percentage deviation (D%) and the hypnotic ($D_H$) and analgesic (Ds) vector components. Finally, the graphical interface displays the datum relative to the increase or decrease of the initial hypnotic and analgesic concentrations, providing the anaesthesiologist with the datum relative to the target hypnotic and analgesic concentrations useful to bring the patient back to the optimal anaesthesia zone (OAZ) defined by the two-dimensional matrix.

According to a preferred embodiment, the graphical interface also allows the two-dimensional matrix and the patient's position (B) with respect to the optimal anaesthesia zone (OAZ) to be visually monitored for variations.

A further object of the present invention is a computer program configured to perform the method of the invention when performed by a computer.

It should be noted that the computer program may perform the steps of the inventive method using generic data management functions.

Those skilled in the art understand that all or some of the steps for implementing the method may be performed by hardware correlated to the program instructions; the program may be stored in a computer-readable storage medium such as one or more computer-readable instructions or computers.

"Storage medium" as referred to herein may refer to media capable of storing information or instructions which can be activated or performed by one or more processors. For example, a storage medium may comprise a database for storing instructions or information readable by a microprocessor.

If performed, the computer-readable program instructions may allow a computing platform to perform one or more actions. According to a preferred embodiment, the computing platform is a software of a drug delivery machine 103.

According to a preferred embodiment, the computer on which the program is performed is in signal communication with a memory unit configured to receive and record data; according to an alternative embodiment the computer on which the program is performed is in signal communication with sensors configured for measuring the bispectral index ($P_{BIS}$) and mean arterial pressure ($P_{MAP}$) of a patient.

A further subject of the present invention is a Proportional-Integral-Derivative (PID) controller configured to implement the method of the invention.

The method of the invention can be implemented with the use of conventional PID controllers, configured to maintain or adjust the parameters of a process to a predetermined value as a function of the deviation thereof from a predetermined range, by means of correction devices known to those skilled in the art.

According to one example application, the PID controller can be integrated into a system for controlling hypnotic and/or analgesic concentrations during anaesthesia, and configured to be in signal communication with

- a memory unit configured to receive and record data;

- at least one sensor for measuring the hypnotic and/or analgesic concentrations in the patient's blood, in signal communication with the memory unit;

- a drug delivery unit configured to deliver at least one hypnotic and one analgesic.

Thus integrated, the PID controller may be configured to perform the inventive method and generate at least one signal based on the difference between the target hypnotic and/or analgesic concentrations obtained by performing the inventive method and the hypnotic and/or analgesic concentrations measured in the patient's blood which, if applied to the delivery unit, would cause the release of hypnotic and/or analgesic to stabilize the hypnotic and/or analgesic concentrations in the patient's blood to the target concentration.

The PID control system may be configured, for example, to implement the method of the invention in fully intravenous delivery systems.

The method of the invention can be of considerable support to anaesthesiologists, to help them define a strategy for modulating and controlling the concentrations of anaesthetic agents during anaesthesia; it should be noted in any case that the choice to administer the target hypnotic and/or anaesthetic concentrations obtained by processing the patient's $P_{BIS}$ and $P_{MAP}$ data, to deviate in part or in whole from the strategy proposed by the method of the invention, remains the responsibility of the doctor, by virtue of the clinical experience acquired in the field.

**EXAMPLE**

By way of illustration and not limitation, an exemplary embodiment of the method of the invention is given below.

*EXAMPLE 1*

**1. Algorithm response test**

The following test demonstrates how the calculations underlying the method of the invention would be performed in clinical practice for the control of hypnotic and analgesic concentrations.

For this purpose, a number of previously recorded non-simulated clinical anaesthesia situations were retrospectively analyzed.

For each anaesthetic procedure analyzed, the maintenance step was taken into account, i.e., the time period between the incision of the skin and the beginning of the surgical suture.

The data were extracted from anaesthesia records as individual monitoring points, characterized by the combination of $P_{BIS}$ and $P_{MAP}$ values, together with the corresponding concentrations of two anaesthetic agents: Propofol (hypnotic) and Remifentanil (opioid analgesic). The concentrations indicated are to be understood as the concentrations expected at the site of action.

For the purposes of the test, the matrix was defined as follows:

- MAP dimension: 0-150 mmHg;

- BIS dimension: 0-100;

- OAZ: between MAP values 65-85 mmHg and BIS values 40-60;

- point (A) in OAZ: MAP equal to 75 mmHg and BIS equal to 50.

Table 1 collects 3 monitoring points characterized by percentage deviations (D%) between 1% and 8%.

The three points (e.g., 1-3) fall within the optimal anaesthesia zone (OAZ) and within the permitted variation limits of percentage deviation (D%) and deviations from hypnosis ($D_H$) and from analgesia ($D_S$). When applied to such monitoring points, the algorithm performed neither hypnotic concentration nor opioid concentration corrections.

**1.1 Area (1) - Increasing BIS and MAP**

Area (1) corresponds to a condition of light anaesthesia, both in terms of hypnosis and in terms of analgesia. In this area the algorithm underlying the method of the invention intervenes to correct both the analgesia components, to bring the patient to an optimal anaesthetic condition.

Table 2 collects 3 monitoring points used for the test and localized in area (1) of the matrix; when applied to these monitoring points, the algorithm intervenes on the calculation of both the hypnotic and opioid concentration, determining an increase delta for each anaesthetic agent, where necessary.

In the case of monitoring point numbers 2 and 3, characterized by a small difference in the $P_{BIS}$ value, a percentage deviation of 29.3% and 26.0% (D%), respectively, was recorded.

Although minimal, this difference results in a different orientation of the vectors (V) describing the distance of each monitoring point (the patient's position (B)) from the center (A) of the optimal anaesthesia zone (OAZ). In an angular system, such as that of the matrix used in the method, a small difference in vector orientation changes the distance of the patient's position (B) from point (A) of the optimal anaesthesia zone (OAZ), and consequently, changes the percentage contribution of each vector on the maximum distance from the center (C).

## 1.2 Area (2) - Decreasing BIS and MAP

Area (2) corresponds to a situation of deep anaesthesia, both in terms of hypnotic component and in terms of analgesic component. In this area, the algorithm underlying the inventive method proposes to lighten both of the anaesthesia components, to bring the patient to an optimal anaesthesia condition.

Table 3 includes 3 monitoring points within area (2) which were used for the purposes of the test. Only one is the monitoring point for which the algorithm performed a simultaneous calculation operation on both anaesthesia components, reducing both the Propofol concentration and the Remifentanil concentration.

For the remaining 2 monitoring points, the algorithm intervened on the calculation of only one of the two drugs: the concentration of Propofol was reduced in both cases.

## 1.3 Area (3) - decreasing BIS and increasing MAP.

Area (3) corresponds to a deep anaesthesia zone in terms of hypnosis, in which the analgesic component is insufficient to control the stress response to trauma.

Table 4 collects the 3 monitoring points, located in area (3) of the matrix, used for the purposes of the test.

For point 1, the control variable which activates the execution of the algorithm is $P_{BIS}$ and, in this sense, a calculation intervention on the Propofol concentration is recorded.

For point number 2, an intervention was recorded on both anaesthetic agents: a decrease in Propofol concentration and an increase in Remifentanil concentration. In three other cases, the control variable outside the optimal range is $P_{MAP}$ and the intervention recorded concerns only the concentration of Remifentanil.

## 1.4 Area (4) - increasing BIS and decreasing MAP

Area (4) corresponds to an anaesthesia situation in which the hypnotic component is insufficient and the MAP values are lower than the optimal anaesthesia zone.

Table 5 includes 3 monitoring points localized in area (4), used for the purposes of the test. The behavior of the inventive method, specifically the algorithm implemented therein, is evident from the analysis of point number 2, where both $P_{BIS}$ and $P_{MAP}$ values are outside the limits allowed by the system; in this case an intervention on the concentration of both hypnotic agents (increasing Propofol and decreasing Remifentanil) was recorded.

In the other two points, the algorithm stops and does not intervene on the calculation of the Propofol concentration, since the deviation from hypnosis (DH) is less than 7.5%.

## 2. Conclusions

The test allowed to show how the calculations underlying the inventive method are useful in defining a strategy to modulate the concentration of anaesthetic agents in a context of non-simulated data. The calculation interventions of the system were always consistent with the clinical situation recorded in the documents.

| OAZ | BIS | MAP | Propofol mcg/mL | Remifentanil ng/mL | D% | $D_H$ | $D_s$ | Δ Propofol mcg/mL | Δ Remifentanil ng/mL |
|---|---|---|---|---|---|---|---|---|---|
| ex 1 | 49 | 75 | 2.8 | 4.0 | 1.3 | -1.3 | 0.0 | 0.0 | 0.0 |
| ex 2 | 46 | 71 | 3.7 | 6.0 | 8.0 | -4.8 | -3.2 | 0.0 | 0.0 |
| ex 3 | 54 | 77 | 1.5 | 5.0 | 8.0 | 6.0 | 2.0 | 0.0 | 0.0 |

**Table 1**

| Area 1 | BIS | MAP | Propofol mcg/mL | Remifentanil ng/mL | D% | $D_H$ | $D_S$ | Δ Propofol mcg/mL | Δ Remifentanil ng/mL |
|---|---|---|---|---|---|---|---|---|---|
| ex 1 | 75 | 76 | 2.0 | 1.0 | 50.0 | 48.7 | 1.3 | 1.5 | 0.0 |
| ex 2 | 60 | 97 | 3.0 | 4.0 | 29.3 | 11.9 | 17.4 | 0.4 | 1.0 |
| ex 3 | 63 | 83 | 3.3 | 4.0 | 26.0 | 18.4 | 7.6 | 0.6 | 0.5 |

**Table 2**

| Area 2 | BIS | MAP | Propofol mcg/mL | Remifentanil ng/mL | D% | $D_H$ | $D_S$ | Δ Propofol mcg/mL | Δ Remifentanil ng/mL |
|---|---|---|---|---|---|---|---|---|---|
| ex 1 | 20 | 74 | 5.0 | 4.0 | 60.0 | -58.7 | -1.3 | -2.9 | 0.0 |
| ex 2 | 25 | 72 | 2.5 | 3.5 | 50.0 | -46.3 | -3.7 | -1.2 | 0.0 |
| ex 3 | 28 | 61 | 2.4 | 4.0 | 44.0 | -30.9 | -13.1 | -0.7 | -0.5 |

**Table 3**

| Area 3 | BIS | MAP | Propofol mcg/mL | Remifentanil ng/mL | D% | $D_H$ | $D_S$ | Δ Propofol mcg/mL | Δ Remifentanil ng/mL |
|---|---|---|---|---|---|---|---|---|---|
| ex 1 | 29 | 77 | 2.8 | 2.0 | 42.0 | -39.5 | 2.5 | -1.1 | 0.0 |
| ex 2 | 34 | 83 | 2.8 | 3.5 | 32.0 | -24.0 | 8.0 | -0.7 | 0.5 |
| ex 3 | 45 | 91 | 3.0 | 2.0 | 21.3 | -6.8 | 14.5 | 0.0 | 0.9 |

**Table 4**

| Area 4 | BIS | MAP | Propofol mcg/mL | Remifentanil ng/mL | D% | D_H | D_S | Δ Propofol mcg/mL | Δ Remifentanil ng/mL |
|---|---|---|---|---|---|---|---|---|---|
| ex 1 | 53 | 48 | 3.0 | 3.0 | 36.0 | 5.1 | -30.9 | 0.0 | -0.9 |
| ex 2 | 65 | 60 | 2.0 | 5.0 | 30.0 | 18.0 | -12.0 | 0.5 | -0.6 |
| ex 3 | 55 | 62 | 2.5 | 2.5 | 17.3 | 6.3 | -11.0 | 0.0 | -0.3 |

**Table 5**

Note that the values of D, $D_H$ and $D_S$ reported in Tables 1 to 5 are expressed in absolute value.

## *EXAMPLE 2*

**1. Baseline clinical situation:** $P_{BIS}$ = 75, $P_{MAP}$ = 130 mmHg
PROPOFOL [Ce]: 3.0 mcg/mL
REMIFENTANIL [Ce]: 5.0 ng/mL

## 2. Calculation of the Percentage Deviation (D%).

The two-dimensional matrix is described as a Cartesian plane defined, on the abscissa, by MAP (0-150 mmHg), and on the ordinate, by BIS (0-100) where the dimensions MAP and BIS orthogonally intersect each other at point zero (0;0).

The matrix is further defined by an ISO-MAP axis, at a MAP value of 75 mmHg, and by an ISO-BIS axis, at a BIS value of 50; the center of the Cartesian plane corresponds to BIS = 50 and MAP = 75 mmHg. The center of the Cartesian plane corresponds to point (A) of the optimal anaesthesia zone (OAZ).

The distance between the points on the plane is calculated using the Pythagorean Theorem.

In the example (Figure 6) segment A-B identifies the hypotenuse, A-E and B-E the two sides of the same triangle (catheters). Similarly, the A-C coordinates identify the hypotenuse, A-D and C-D the two sides of the corresponding triangle.

The value of point B on the abscissa corresponds to $P_{MAP}$; the value of point B on the ordinate corresponds to $P_{BIS}$.

Accordingly, the algorithm uses Formula 4
(**AB**: $\sqrt{(AE^2 + BE^2)}$ to determine the distance between points A and B; Formula 5
(**AC**: $\sqrt{(AD^2 + CD^2)}$ to determine the distance between points A and C.

| DATUM | VECTOR CALCULATION: Pythagorean Theorem | CALCULATION OF D% |
|---|---|---|
| AE: 130-75 = 55 BE: 75-50 = 25 AD: 150-75 = 75 CD: 84-50 = 34 | $\mathbf{AB:} \sqrt{55^2 + 25^2} = 60,4$ $\mathbf{AC:} \sqrt{75^2 + 34^2} = 82,4$ | $\boldsymbol{D}\% = \dfrac{A - B}{A - C} * 100 = 73,3\%$ |

## 3. Calculation of the hypnotic (H) and analgesic (S) vector components.

The algorithm analyzes the distance of the head (A) of the vector (A-B) from the orthogonal axes defined by the coordinates BIS 50 - the ISO-BIS axis - and MAP 75 mmHg - the ISO-MAP axis.

The distance of the head (A) of the vector AB from the ISO-BIS axis is indicated as component "H" and is calculated in accordance with Formula 9

$$(\mathbf{H} = P_{BIS} - 50);$$

the distance of the head (A) from the ISO-MAP axis is indicated as component "S" and is calculated in accordance with Formula 12

$$(\mathbf{S} = [(P_{MAP} - 75)/150]*100).$$

This calculation method satisfies the need to prevent that the greater width of the MAP dimension results in a greater weight of this variable in the vectorial calculation.

The two vector components - **"H"** and **"S"** - are used to quantify the fraction of D% to be attributed to the hypnotic component - referred to as $\mathbf{D_H}$ - and to be attributed to the analgesic component - referred to as $\mathbf{D_S}$ - according to the following expressions:

$$\text{Formula 7: } \mathbf{D_H} = [H/(|H|+|S|)]*D\%$$

$$\text{Formula 14: } \mathbf{D_S} = [S/(|H|+|S|)]*D\%$$

$\mathbf{D_H}$ and $\mathbf{D_S}$ are the percentages which quantify the intervention to be performed on the hypnotic agent and analgesic agent each time the patient exits the OAZ.

| DATUM | CALCULATION OF $D_H$ and $D_S$ |
|---|---|
| **D%:** (A-B/A-C)*100 = 73.3<br>**H:** 75 - 50 = 25.0<br>**S:** [(130 - 75)/150]*100 = 36.7 | $\mathbf{D_H}$: (25/(\|25\| + \|37\|)*73.3 = 29.7 %<br><br>$\mathbf{D_S}$: (37/(\|25\| + \|37\|)*73.3 = 43.6 % |

## 4. Computational calculation of titration.

This step of the algorithm integrates vector analysis output - $\mathbf{D_H}$ and $\mathbf{D_S}$ - with the concentration data coming from the controlled infusion system (TCI) to define the useful target concentrations of Propofol and Remifentanil to bring the patient into the OAZ. The system is designed to intervene on the anaesthetic concentrations and cannot manage the dosage or infusion rate directly. The drug concentrations are to be understood as the action site concentrations (Ce) as provided by the controlled infusion system (TCI).

The equation for quantifying the intervention on the anaesthetic agents is different if the therapeutic goal is an increase or decrease in drug concentrations.

In this example embodiment, the drugs used are Remifentanil and Propofol; below are the equations to calculate an increase in the concentration thereof:

$$\text{Formula 16: } [c_{\text{target}}]_{\uparrow Propofol} = [c_{in}]_{Propofol} + (D_H * 3)$$

$$\text{Formula 17: } [c_{\text{target}}]_{\uparrow Remifentanil} = [c_{in}]_{Remifentanil} + (D_S * 6)$$

The equations for calculating a decrease in the concentration thereof are shown below:

$$\text{Formula 19: } [c_{\text{target}}]_{\downarrow Propofol} = [c_{in}]_{Propofol} + ([c_{in}]_{Propofol} * [-D_H])$$

$$\text{Formula 20: } [c_{\text{target}}]_{\downarrow Remifentanil} = [c_{in}]_{Remifentanil} ([c_{in}]_{Remifentanil} * [-D_S])$$

| Vector analysis output | $C_{in}$ | Effective concentration at the site of action | Target concentration |
|---|---|---|---|
| DH: 29.7% | 3 µg/mL | PROPOFOL [Ce]: 3.0 mcg/mL | PROPOFOL [Ce]: 3.9 mcg/mL |
| DS: 43.6% | 5 ng/mL | REMIFENTANIL [Ce]: 5.0 ng/mL | REMIFENTANIL [Ce]: 7.6 ng/mL |

REFERENCES

1. Lundy JS. Balanced anaesthesia. Minn Med 1926; 9: 399.

2. P. H. Tonner. Balanced anaesthesia today. Best Practice & Research Clinical Anaesthesiology 2005, 19(3): 475-484.

3. William R Greco et al. The Search for Synergy: A Critical review from a Response Surface Prospective. PHARMACOLOGICAL REVIEWS 1995, 47(2): 331-385.

4. Jaap Vuyk et al. Propofol Anaesthesia and Rational Opioid Selection: Determination of Optimal EC50-EC95 Propofol-Opioid Concentrations that Assure Adequate Anaesthesia and a Rapid Return of Consciousness. Anaesthesiology 1997, 87(12):1549-1562.

5. an F. A. Hendrickx et al. Is Synergy the Rule? A Review of Anaesthetic Interactions Producing Hypnosis and Immobility. Anaesthesia & Analgesia: August 2008 - Volume 107 - Issue 2 - p 494-506.

6. Gurman GM. Assessment of depth of general anaesthesia. Observations on processed EEG and spectral edge frequency. Int J Clin Monit Comput 1994; 11: 185-9.

7. Daniel I. Sessler et al. Hospital Stay and Mortality Are Increased in Patients Having a "Triple Low" of Low Blood Pressure, Low Bispectral Index, and Low Minimum Alveolar Concentration of Volatile Anaesthesia. Anaesthesiology 2012; 116:1195-203.

8. Gan TJ, Glass PS, Windsor A, Payne F, Rosow C, Sebel P, Manberg P. Bispectral index monitoring allows faster emergence and improved recovery from propofol, alfentanil, and nitrous oxide anaesthesia. BIS Utility Study Group. Anaesthesiology 1997; 87: 808-15.

**Claims**

1. Method for the control of hypnotic and analgesic concentrations of an anaesthetic composition comprising the following steps:

    - supplying a memory unit configured to receive and record data;
    - supplying at least one data processing unit (101), in signal communication with the memory unit and configured to process and record data in the memory unit;
    - providing the bispectral index ($P_{BIS}$) and the mean arterial pressure ($P_{MAP}$) of a patient treated with a first anaesthetic composition comprising an initial hypnotic concentration and an initial analgesic concentration, and storing the patient's $P_{BIS}$ and $P_{MAP}$ data in said memory unit;

    **the method being characterized in that it comprises the following steps:**

    - providing a two-dimensional matrix defined, on the abscissa, by a MAP dimension relative to the variation of Mean Arterial Pressure and on the ordinate by a BIS dimension relative to the variation of the Bispectral Index;
    - defining in the matrix an optimal anaesthesia zone (OAZ) located between MAP values of 65 and 110 mmHg and BIS values of 40 and 60;
    - defining in the matrix an ISO-MAP axis and an ISO-BIS axis, orthogonal to ISO-MAP, whose intersection defines a point (A) of the optimal anaesthesia zone (OAZ);

- localizing in the matrix the values of the $P_{BIS}$ and $P_{MAP}$ data, to define a position (B) of the patient in the matrix with respect to the optimal anaesthesia zone (OAZ);

- providing an algorithm resident in said processing unit, configured to quantify the deviation of the patient's position (B) with respect to the optimal anaesthesia zone (OAZ) and transforming the deviation into a quantitative variation of the initial hypnotic concentration and the initial analgesic concentration of the first anaesthetic composition to define a target hypnotic concentration and a target analgesic concentration of a second anaesthetic composition;

- processing the patient's $P_{BIS}$ and $P_{MAP}$ data using said algorithm to obtain the target hypnotic concentration and the target anaesthetic concentration of the second anaesthetic composition, the step of processing the patient's $P_{BIS}$ and $P_{MAP}$ data comprising the sub-steps of:

- quantifying the deviation of the patient's position (B) with respect to point (A) of the optimal anaesthesia zone (OAZ) by means of a vectorial calculation, the sub-step of quantifying comprising the sub-steps of

- defining a vector (V) passing through point (A) of the optimal anaesthesia zone (OAZ) through the patient's position (B) and
- calculating a percentage deviation (D%) of the patient's position (B) with respect to point (A) of the optimal anaesthesia zone (OAZ) on the maximum deviation (C) of the MAP and BIS values along the vector (V),

- decomposing the deviation of the patient's position (B) with respect to point (A) of the optimal anaesthesia zone (OAZ) into a deviation from hypnosis ($D_H$) and/or into a deviation from analgesia ($D_S$) with respect to point (A) of the optimal anaesthesia zone (OAZ), and
- calculating an increase or decrease in the initial hypnotic and/or analgesic concentration of the first anaesthetic composition based on said deviations ($D_H$; $D_S$), said sub-step of calculating comprising the sub-step of

- modifying the hypnotic and analgesic concentration if the percentage deviation (D%) of the patient's position (B) with respect to point (A) of the optimal anaesthesia zone (OAZ) is greater than 10%,

- generating, by means of the algorithm, a datum relative to the target hypnotic and analgesic concentrations of the second anaesthetic composition, said datum being configured to induce the processing unit (101) to generate a receivable signal from a graphical interface device (102) configured to display target hypnotic and sedative concentrations processed by the algorithm, and/or from a machine configured for the delivery of one or more drugs (103).

2. The method according to claim 1, wherein the step of defining the optimal anaesthesia zone (OAZ) involves localizing the optimal anaesthesia zone (OAZ) between MAP values of 65 and 85 mmHg and BIS values of 40 and 60.

3. The method according to claim 1 or 2, wherein the matrix is defined by a MAP variation between 0 and 150 mmHg and by a BIS variation between 0 and 100, where the MAP and BIS dimensions orthogonally intersect each other at the zero point (0; 0).

4. The method according to any one of claims from 1 to 3, wherein point (A) is defined by the intersection of the ISO-MAP axis at a MAP value of 75 mmHg and of the ISO-BIS axis at a BIS value of 50.

5. The method according to anyone of claims from 1 to 4, wherein the percentage deviation (D%) of the patient's position (B) with respect to point (A) of the optimal anaesthesia zone (OAZ), on the maximum deviation (C) of the MAP and BIS values along the vector (V), is obtained with the expression:

$$D\% = \frac{A-B}{A-C} * 100$$

6. The method according to any one of claims from 1 to 5, wherein the sub-step of decomposing the deviation of the patient's position (B) with respect to point (A) of the optimal anaesthesia zone (OAZ) comprises a sub-step of calculating the deviation from hypnosis ($D_H$) by applying the percentage deviation (D%) of the patient's position (B) with respect to point (A) of the optimal anaesthesia zone (OAZ) at a hypnotic coefficient (H') obtained by dividing a hypnotic vector component (H), measured as the distance between the patient's position (B) and the ISO-BIS axis, by the sum of the hypnotic vector component (H) and an analgesic vector component (S) measured as the distance between the patient's position (B) and the ISO-MAP axis.

7. The method according to any one of claims from 1 to 6, wherein the sub-step of calculating an increase or decrease in the hypnotic and/or analgesic initial concentration comprises the following sub-steps:

- changing the hypnotic concentration if the deviation from hypnosis ($D_H$) is greater than 7.5%; and
- changing the analgesic concentration if the deviation from analgesia ($D_S$) is greater than 7.5%.

8. The method according to any one of claims from 1 to 7, wherein the sub-step of calculating an increase or decrease in the hypnotic and/or analgesic initial concentration comprises the following sub-steps:

- modifying the hypnotic concentration if the percentage deviation (D%) of the patient's position (B) from point (A) of the optimal anaesthesia zone (OAZ) is greater than 10% and, at the same time, the deviation from hypnosis ($D_H$) is greater than 7.5%;
- modifying the analgesic concentration if the percentage deviation (D%) of the patient's position (B) from point (A) of the optimal anaesthesia zone (OAZ) is greater than 10% and, at the same time, the deviation from analgesia ($D_S$) is greater than 7.5%.

9. The method according to any one of claims from 1 to 8, wherein the increase in the hypnotic and analgesic initial concentration of the first anaesthetic composition as a function of said deviations ($D_H$; $D_S$) is obtained with the expression

$$[c_{target}]\uparrow = [c_{in}] + \{D_{H/S} * ([c_{max}] - [c_{in}])\}$$

10. The method according to any one of claims from 1 to 9, wherein the decrease in the hypnotic and analgesic initial concentration of the first anaesthetic composition as a function of said deviations ($D_H$; $D_S$) is obtained with the expression

$$[c_{target}]\downarrow = [c_{in}] - (D_{H/S} * [c_{in}])$$

11. The method according to any one of claims from 1 to 10, further comprising the step of:

- generating, by means of the processing unit (101), a signal as a function of the datum relative to the target hypnotic and analgesic concentrations and sending the signal to a graphical interface device (102) configured to display the target hypnotic and sedative concentrations processed by the algorithm;
and/or
- generating, by means of the processing unit (101), a signal as a function of the datum relative to the target hypnotic and analgesic concentrations to be used by a machine configured to deliver one or more drugs (103) as a function of the target hypnotic and sedative concentrations processed by the algorithm.

12. The method according to claim 11, further comprising the step of approving the datum relative to the target hypnotic and analgesic concentration of the second anaesthetic composition by the anaesthesiologist by means of the graphical interface.

13. Computer program configured to perform the method according to any one of the claims from 1 to 11 when performed by a computer.

14. PID controller configured to implement the method according to any one of the claims from 1 to 11.

**Patentansprüche**

1. Verfahren zur Regelung von hypnotischen und analgetischen Konzentrationen einer anästhetischen Zusammensetzung, umfassend die folgenden Schritte:

- Bereitstellen einer Speichereinheit, die dazu konfiguriert ist, Daten zu empfangen und aufzuzeichnen;
- Bereitstellen mindestens einer Datenverarbeitungseinheit (101), die in Signalkommunikation mit der Speichereinheit steht und dazu konfiguriert ist, Daten in der Speichereinheit zu verarbeiten und aufzuzeichnen;
- Bereitstellen des bispektralen Indexes ($P_{BIS}$) und des mittleren arteriellen Drucks ($P_{MAP}$) eines Patienten, der mit einer ersten anästhetischen Zusammensetzung behandelt wird, die eine anfängliche hypnotische Konzentration und eine anfängliche analgetische Konzentration umfasst, und Speichern der $P_{BIS}$- und $P_{MAP}$-Daten des Patienten in der besagten Speichereinheit;

**wobei das Verfahren dadurch gekennzeichnet ist, dass es die folgenden Schritte umfasst:**

- Bereitstellen einer zweidimensionalen Matrix, dieauf der Abszisse durch eine MAP-Dimension relativ zur Variation des mittleren arteriellen Drucks und auf der Ordinate durch eine BIS-Dimension relativ zur Variation des bispektralen Indexes definiert ist;
- Definieren einer optimalen Anästhesiezone (OAZ) in der Matrix, die zwischen PMAP-Werten von 65 und 110 mmHg und BIS-Werten von 40 und 60 liegt;
- Definieren einer ISO-MAP-Achse und einer ISO-BIS-Achse in der Matrix, die orthogonal zur ISO-MAP-Achse ist, und deren Schnittpunkt einen Punkt (A) der optimalen Anästhesiezone (OAZ) definiert;
- Lokalisieren der Werte der $P_{BIS}$- und $P_{MAP}$-Daten in der Matrix, um eine Position (B) des Patienten in der Matrix in Bezug auf die optimale Anästhesiezone (OAZ) zu definieren;
- Bereitstellen eines in der besagten Verarbeitungseinheit residenten Algorithmus, der dazu konfiguriert ist, die Abweichung der Position (B) des Patienten in Bezug auf die optimale Anästhesiezone (OAZ) zu quantifizieren und die Abweichung in eine quantitative Variation der anfänglichen hypnotischen Konzentration und der anfänglichen analgetischen Konzentration der ersten anästhetischen Zusammensetzung umzuwandeln, um eine hypnotische Zielkonzentration und eine analgetische Zielkonzentration einer zweiten anästhetischen Zusammensetzung zu definieren;
- Verarbeiten der $P_{BIS}$- und $P_{MAP}$-Daten des Patienten unter Verwendung des besagten Algorithmus, um die hypnotische Zielkonzentration und die analgetische Zielkonzentration der zweiten anästhetischen Zusammensetzung zu erhalten, wobei der Schritt des Verarbeitens der $P_{BIS}$- und $P_{MAP}$-Daten des Patienten die folgenden Unterschritte umfasst:

  ◦ Quantifizieren der Abweichung der Position (B) des Patienten in Bezug auf den Punkt (A) der optimalen Anästhesiezone (OAZ) mittels einer vektoriellen Berechnung, wobei der Unterschritt des Quantifizierens die Unterschritte umfasst:

    ▪ Definieren eines Vektors (V), der durch den Punkt (A) der optimalen Anästhesiezone (OAZ) durch die Position (B) des Patienten verläuft, und
    ▪ Berechnen einer prozentualen Abweichung (D%) der Position (B) des Patienten in Bezug auf den Punkt (A) der optimalen Anästhesiezone (OAZ) auf der maximalen Abweichung (C) der PMAP- und BIS-Werte entlang des Vektors (V),

  ◦ Zerlegen der Abweichung der Position (B) des Patienten in Bezug auf den Punkt (A) der optimalen Anästhesiezone (OAZ) in eine Abweichung von der Hypnose ($D_H$) und/oder in eine Abweichung von der Analgesie (Ds) in Bezug auf den Punkt (A) der optimalen Anästhesiezone (OAZ), und
  ◦ Berechnen einer Erhöhung oder Verringerung der anfänglichen hypnotischen und/oder analgetischen Konzentration der ersten anästhetischen Zusammensetzung basierend auf den besagten Abweichungen ($D_H$; Ds), wobei der besagte Unterschritt des Berechnens den Unterschritt umfasst:

    ▪ Modifizieren der hypnotischen und analgetischen Konzentration, wenn die prozentuale Abweichung (D%) der Position (B) des Patienten in Bezug auf den Punkt (A) der optimalen Anästhesiezone (OAZ) größer als 10 % ist,

- Erzeugen, mittels des Algorithmus, eines Datums relativ zu den hypnotischen und analgetischen Zielkonzentrationen der zweiten anästhetischen Zusammensetzung, wobei das besagte Datum dazu konfiguriert ist, die Verarbeitungseinheit (101) zu veranlassen, ein empfangbares Signal von einer grafischen Schnittstellenvorrichtung (102), die dazu konfiguriert ist, durch den Algorithmus verarbeitete hypnotische und sedative Zielkonzentrationen anzuzeigen, und/oder von einer Maschine, die für die Verabreichung eines oder mehrerer Medikamente (103) konfiguriert ist, zu erzeugen.

**2.** Verfahren nach Anspruch 1, wobei der Schritt des Definierens der optimalen Anästhesiezone (OAZ) das Lokalisieren der optimalen Anästhesiezone (OAZ) zwischen MAP-Werten von 65 und 85 mmHg und BIS-Werten von 40 und 60 umfasst.

**3.** Verfahren nach Anspruch 1 oder 2, wobei die Matrix durch eine PMAP-Variation zwischen 0 und 150 mmHg und durch eine BIS-Variation zwischen 0 und 100 definiert ist, wobei sich die MAP- und BIS-Dimensionen orthogonal am Nullpunkt (0; 0) schneiden.

**4.** Verfahren nach einem der Ansprüche 1 bis 3, wobei der Punkt (A) durch den Schnittpunkt der ISO-MAP-Achse bei einem MAP-Wert von 75 mmHg und der ISO-BIS-Achse bei einem BIS-Wert von 50 definiert ist.

**5.** Verfahren nach einem der Ansprüche 1 bis 4, wobei die prozentuale Abweichung (D%) der Position (B) des Patienten in Bezug auf den Punkt (A) der optimalen Anästhesiezone (OAZ), auf der maximalen Abweichung (C) der MAP- und BIS-Werte entlang des Vektors (V), mit dem Ausdruck:

$$D\% = \frac{A - B}{A - C} * 100$$

**6.** Verfahren nach einem der Ansprüche 1 bis 5, wobei der Unterschritt des Zerlegens der Abweichung der Position (B) des Patienten in Bezug auf den Punkt (A) der optimalen Anästhesiezone (OAZ) einen Unterschritt des Berechnens der Abweichung von der Hypnose ($D_H$) umfasst, indem die prozentuale Abweichung (D%) der Position (B) des Patienten in Bezug auf den Punkt (A) der optimalen Anästhesiezone (OAZ) auf einen hypnotischen Koeffizienten (H') angewendet wird, der erhalten wird, indem eine hypnotische Vektorkomponente (H), gemessen als der Abstand zwischen der Position (B) des Patienten und der ISO-BIS-Achse, durch die Summe der hypnotischen Vektorkomponente (H) und einer analgetischen Vektorkomponente (S), gemessen als der Abstand zwischen der Position (B) des Patienten und der ISO-MAP-Achse, geteilt wird.

**7.** Verfahren nach einem der Ansprüche 1 bis 6, wobei der Unterschritt des Berechnens einer Erhöhung oder Verringerung der anfänglichen hypnotischen und/oder analgetischen Konzentration die folgenden Unterschritte umfasst:

- Ändern der hypnotischen Konzentration, wenn die Abweichung von der Hypnose ($D_H$) größer als 7,5% ist; und
- Ändern der analgetischen Konzentration, wenn die Abweichung von der Analgesie (Ds) größer als 7,5% ist.

**8.** Verfahren nach einem der Ansprüche 1 bis 7, wobei der Unterschritt des Berechnens einer Erhöhung oder Verringerung der anfänglichen hypnotischen und/oder analgetischen Konzentration die folgenden Unterschritte umfasst:

- Modifizieren der hypnotischen Konzentration, wenn die prozentuale Abweichung (D%) der Position (B) des Patienten von Punkt (A) der optimalen Anästhesiezone (OAZ) größer als 10 % ist und gleichzeitig die Abweichung von der Hypnose ($D_H$) größer als 7,5% ist;
- Modifizieren der analgetischen Konzentration, wenn die prozentuale Abweichung (D%) der Position (B) des Patienten von Punkt (A) der optimalen Anästhesiezone (OAZ) größer als 10 % ist und gleichzeitig die Abweichung von der Analgesie (Ds) größer als 7,5% ist.

**9.** Verfahren nach einem der Ansprüche 1 bis 8, wobei die Erhöhung der anfänglichen hypnotischen und analgetischen Konzentration der ersten anästhetischen Zusammensetzung als Funktion der besagten Abweichungen ($D_H$; Ds) mit dem Ausdruck

$$[c_{target}]\uparrow = [c_{in}] + \{D_{H/S} * ([c_{max}] - [c_{in}])\}$$

erhalten wird.

**10.** Verfahren nach einem der Ansprüche 1 bis 9, wobei die Verringerung der anfänglichen hypnotischen und analgetischen Konzentration der ersten anästhetischen Zusammensetzung als Funktion der besagten Abweichungen ($D_H$; Ds) mit dem Ausdruck

$$[c_{target}]\downarrow = [c_{in}] - (D_{H/S} * [c_{in}])$$

erhalten wird.

11. Verfahren nach einem der Ansprüche 1 bis 10, ferner umfassend den Schritt:

- Erzeugen, mittels der Verarbeitungseinheit (101), eines Signals als Funktion des Datums relativ zu den hypnotischen und analgetischen Zielkonzentrationen und Senden des Signals an eine grafische Schnittstellenvorrichtung (102), die dazu konfiguriert ist, die durch den Algorithmus verarbeiteten hypnotischen und sedativen Zielkonzentrationen anzuzeigen; und/oder

- Erzeugen, mittels der Verarbeitungseinheit (101), eines Signals als Funktion des Datums relativ zu den hypnotischen und analgetischen Zielkonzentrationen, das von einer Maschine verwendet werden soll, die dazu konfiguriert ist, ein oder mehrere Medikamente (103) als Funktion der durch den Algorithmus verarbeiteten hypnotischen und sedativen Zielkonzentrationen zu verabreichen.

12. Verfahren nach Anspruch 11, ferner umfassend den Schritt des Genehmigens des Datums relativ zur hypnotischen und analgetischen Zielkonzentration der zweiten anästhetischen Zusammensetzung durch den Anästhesisten mittels der grafischen Schnittstelle.

13. Computerprogramm, das dazu konfiguriert ist, das Verfahren nach einem der Ansprüche 1 bis 11 durchzuführen, wenn es von einem Computer ausgeführt wird.

14. PID-Regler, der dazu konfiguriert ist, das Verfahren nach einem der Ansprüche 1 bis 11 zu implementieren.

**Revendications**

1. Procédé de contrôle des concentrations hypnotiques et analgésiques d'une composition anesthésique comprenant les étapes suivantes :

- fournir une unité de mémoire configurée pour recevoir et enregistrer des données ;
- fournir au moins une unité de traitement de données (101), en communication de signal avec l'unité de mémoire et configurée pour traiter et enregistrer des données dans l'unité de mémoire;
- fournir l'indice bispectral ($P_{BIS}$) et la pression artérielle moyenne ($P_{MAP}$) d'un patient traité avec une première composition anesthésique comprenant une concentration hypnotique initiale et une concentration analgésique initiale, et stocker les données $P_{BIS}$ et $P_{MAP}$ du patient dans ladite unité de mémoire;

**le procédé étant caractérisé en ce qu'il comprend les étapes suivantes consistant à:**

- fournir une matrice bidimensionnelle définie, en abscisse, par une dimension MAP relative à la variation de la Pression Artérielle Moyenne et en ordonnée par une dimension BIS relative à la variation de l'Indice Bispectral;
- définir dans la matrice une zone d'anesthésie optimale (OAZ) située entre des valeurs de $P_{MAP}$ de 65 et 110 mmHg et des valeurs de BIS de 40 et 60;
- définir dans la matrice un axe ISO-MAP et un axe ISO-BIS, orthogonal à l'axe ISO-MAP, dont l'intersection définit un point (A) de la zone d'anesthésie optimale (OAZ) ;
- localiser dans la matrice les valeurs des données $P_{BIS}$ et $P_{MAP}$, pour définir une position (B) du patient dans la matrice par rapport à la zone d'anesthésie optimale (OAZ);
- fournir un algorithme résidant dans ladite unité de traitement, configuré pour quantifier l'écart de la position (B) du patient par rapport à la zone d'anesthésie optimale (OAZ) et transformer l'écart en une variation quantitative de la concentration hypnotique initiale et de la concentration analgésique initiale de la première composition anesthésique pour définir une concentration hypnotique cible et une concentration analgésique cible d'une seconde composition anesthésique;
- traiter les données $P_{BIS}$ et $P_{MAP}$ du patient en utilisant ledit algorithme pour obtenir la concentration hypnotique cible et la concentration anesthésique cible de la seconde composition anesthésique, l'étape de traitement des données $P_{BIS}$ et $P_{MAP}$ du patient comprenant les sous-étapes consistant à:

  ◦ quantifier l'écart de la position (B) du patient par rapport au point (A) de la zone d'anesthésie optimale (OAZ) au moyen d'un calcul vectoriel, la sous-étape de quantification comprenant les sous-étapes consistant à

    ▪ définir un vecteur (V) passant par le point (A) de la zone d'anesthésie optimale (OAZ) à travers la position (B) du patient et

■ calculer un écart en pourcentage (D%) de la position (B) du patient par rapport au point (A) de la zone d'anesthésie optimale (OAZ) sur l'écart maximal (C) des valeurs de MAP et de BIS le long du vecteur (V),

○ décomposer l'écart de la position (B) du patient par rapport au point (A) de la zone d'anesthésie optimale (OAZ) en un écart par rapport à l'hypnose ($D_H$) et/ou en un écart par rapport à l'analgésie (Ds) par rapport au point (A) de la zone d'anesthésie optimale (OAZ), et

○ calculer une augmentation ou une diminution de la concentration hypnotique et/ou analgésique initiale de la première composition anesthésique sur la base desdits écarts ($D_H$; Ds), ladite sous-étape de calcul comprenant la sous-étape consistant à

■ modifier la concentration hypnotique et analgésique si l'écart en pourcentage (D%) de la position (B) du patient par rapport au point (A) de la zone d'anesthésie optimale (OAZ) est supérieur à 10%,

- générer, au moyen de l'algorithme, une donnée relative aux concentrations hypnotiques et analgésiques cibles de la seconde composition anesthésique, ladite donnée étant configurée pour amener l'unité de traitement (101) à générer un signal recevable provenant d'un dispositif d'interface graphique (102) configuré pour afficher les concentrations hypnotiques et sédatives cibles traitées par l'algorithme, et/ou d'une machine configurée pour l'administration d'un ou plusieurs médicaments (103).

2. Procédé selon la revendication 1, dans lequel l'étape de définition de la zone d'anesthésie optimale (OAZ) implique de localiser la zone d'anesthésie optimale (OAZ) entre des valeurs de MAP de 65 et 85 mmHg et des valeurs de BIS de 40 et 60.

3. Procédé selon la revendication 1 ou 2, dans lequel la matrice est définie par une variation de MAP entre 0 et 150 mmHg et par une variation de BIS entre 0 et 100, où les dimensions MAP et BIS se coupent orthogonalement au point zéro (0; 0).

4. Procédé selon l'une quelconque des revendications 1 à 3, dans lequel le point (A) est défini par l'intersection de l'axe ISO-MAP à une valeur de MAP de 75 mmHg et de l'axe ISO-BIS à une valeur de BIS de 50.

5. Procédé selon l'une quelconque des revendications 1 à 4, dans lequel l'écart en pourcentage (D%) de la position (B) du patient par rapport au point (A) de la zone d'anesthésie optimale (OAZ), sur l'écart maximal (C) des valeurs de MAP et de BIS le long du vecteur (V), est obtenu avec l'expression:

$$D\% = \frac{A - B}{A - C} * 100$$

6. Procédé selon l'une quelconque des revendications 1 à 5, dans lequel la sous-étape de décomposition de l'écart de la position (B) du patient par rapport au point (A) de la zone d'anesthésie optimale (OAZ) comprend une sous-étape de calcul de l'écart par rapport à l'hypnose ($D_H$) en appliquant l'écart en pourcentage (D%) de la position (B) du patient par rapport au point (A) de la zone d'anesthésie optimale (OAZ) à un coefficient hypnotique (H') obtenu en divisant une composante vectorielle hypnotique (H), mesurée comme la distance entre la position (B) du patient et l'axe ISO-BIS, par la somme de la composante vectorielle hypnotique (H) et d'une composante vectorielle analgésique (S) mesurée comme la distance entre la position (B) du patient et l'axe ISO-MAP.

7. Procédé selon l'une quelconque des revendications 1 à 6, dans lequel la sous-étape de calcul d'une augmentation ou d'une diminution de la concentration initiale hypnotique et/ou analgésique comprend les sous-étapes suivantes :

- modifier la concentration hypnotique si l'écart par rapport à l'hypnose ($D_H$) est supérieur à 7,5% ; et
- modifier la concentration analgésique si l'écart par rapport à l'analgésie (Ds) est supérieur à 7,5%.

8. Procédé selon l'une quelconque des revendications 1 à 7, dans lequel la sous-étape de calcul d'une augmentation ou d'une diminution de la concentration initiale hypnotique et/ou analgésique comprend les sous-étapes suivantes:

- modifier la concentration hypnotique si l'écart en pourcentage (D%) de la position (B) du patient par rapport au point (A) de la zone d'anesthésie optimale (OAZ) est supérieur à 10 % et, en même temps, l'écart par rapport à l'hypnose ($D_H$) est supérieur à 7,5 %;
- modifier la concentration analgésique si l'écart en pourcentage (D%) de la position (B) du patient par rapport au point (A) de la zone d'anesthésie optimale (OAZ) est supérieur à 10 % et, en même temps, l'écart par rapport à l'analgésie (Ds) est supérieur à 7,5%.

**9.** Procédé selon l'une quelconque des revendications 1 à 8, dans lequel l'augmentation de la concentration initiale hypnotique et analgésique de la première composition anesthésique en fonction desdits écarts ($D_H$; Ds) est obtenue avec l'expression

$$[c_{target}]\!\uparrow \, = [c_{in}] + \{D_{H/S} * ([c_{max}] - [c_{in}])\}$$

**10.** Procédé selon l'une quelconque des revendications 1 à 9, dans lequel la diminution de la concentration initiale hypnotique et analgésique de la première composition anesthésique en fonction desdits écarts ($D_H$; Ds) est obtenue avec l'expression

$$[c_{target}]\!\downarrow \, = [c_{in}] - (D_{H/S} * [c_{in}])$$

**11.** Procédé selon l'une quelconque des revendications 1 à 10, comprenant en outre l'étape consistant à:

- générer, au moyen de l'unité de traitement (101), un signal en fonction de la donnée relative aux concentrations hypnotiques et analgésiques cibles et envoyer le signal à un dispositif d'interface graphique (102) configuré pour afficher les concentrations hypnotiques et sédatives cibles traitées par l'algorithme; et/ou
- générer, au moyen de l'unité de traitement (101), un signal en fonction de la donnée relative aux concentrations hypnotiques et analgésiques cibles devant être utilisé par une machine configurée pour administrer un ou plusieurs médicaments (103) en fonction des concentrations hypnotiques et sédatives cibles traitées par l'algorithme.

**12.** Procédé selon la revendication 11, comprenant en outre l'étape d'approbation de la donnée relative à la concentration hypnotique et analgésique cible de la seconde composition anesthésique par l'anesthésiste au moyen de l'interface graphique.

**13.** Programme d'ordinateur configuré pour mettre en œuvre le procédé selon l'une quelconque des revendications 1 à 11 lorsqu'il est exécuté par un ordinateur.

**14.** Contrôleur PID configuré pour mettre en œuvre le procédé selon l'une quelconque des revendications 1 à 11.

Figure 1

Figure 2

| | BP | HIGH* | NORMAL | LOW* |
|---|---|---|---|---|
| SEF (Hz) | | | | |
| > 12 | | A | B | C |
| 8 - 12 | | D | IDEAL SITUATION | E |
| < 8 | | F | G | H |

Figure 3

Figure 4

Figure 5

**To rise concentration up:**

$[c_{in}] + \{D_{H/S} *([c_{max}] - [c_{in}])\}$

0        Actual        **max**
         concentration

——— Drug concentration >>———

**To cut concentrations down:**

$[c_{in}] - (D_{H/S} * [c_{in}])$

Figure 6

**BIS** = 75        **MAP** = 130 mmHg

Figure 7

Figure 8

## VECTOR ANALSIS

| | BIS (0-100) | MAP mmHg (0-150) | | FILTERS | |
|---|---|---|---|---|---|
| TARGETS | 50 | 75 | | MAIN FILTER: CUT-OFF = 10% | |
| CURRENT VALUES | 78 | 130 | | EXCESS D₁ | YES |

| | TRIANGLES SIDES | | | | |
|---|---|---|---|---|---|
| | AE | 55,0 | | SECONDARY FILTER: CUT-OFF = 7,5% | |
| | AD | 75,0 | | | |
| | BE | 25,0 | | EXCESS D₁₁ | YES |
| | CD | 34,1 | | EXCESS D₁₂ | YES |

| | | H | S | VECTOR ANALYSIS – FINAL OUTPUT | |
|---|---|---|---|---|---|
| Vector AB | 60,4 | 25,0 | 36,7 | | |
| Vector AC | 82,4 | H' | S' | $D_H\%$ | $D_S\%$ |
| | | 0,4 | 0,6 | | |
| OUTPUT : | D₁ (%) | D₁₁ (%) | D₁₂ (%) | 29,7 | 43,6 |
| | 73,3 | 29,7 | 43,6 | | |

## TITRATION COMPUTATIONAL METHOD

| CURRENT CONCENTRATIONS | | UPWARD | UPWARD | NEW CONCENTRATIONS TARGET | |
|---|---|---|---|---|---|
| PROPOFOL Ce mcg/mL | REMIFENTANIL Ce ng/mL | Δ PROPOFOL Ce mcg/mL | Δ REMIFENTANIL Ce ng/mL | PROPOFOL mcg/mL | REMIFENTANIL ng/mL |
| 3,0 | 5,0 | 0,9 | 2,6 | 3,9 | 7,6 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012095437 A1

### Non-patent literature cited in the description

- **J. BRUHN** ; **P. S. MYLES** ; **R. SNEYD** ; **M. M. R. F. STRUYS**. Depth of anaesthesia monitoring: what's available, what's validated and what's next?. *BJA: British Journal of Anaesthesia*, July 2006, vol. 97 (1), 85-94, https://doi.org/10.1093/bja/ael120
- **KAI KUCK**. Periopera6ve Noninvasive Blood Pressure Monitoring. *Anaesth Analg*, 2018, vol. 127, 408-11
- **KARSTEN BARTELS**. *Blood Pressure Monitoring for the Anaesthesiologist: A Practical Review. Anaesth Analg*, 2016, vol. 122, 1866-79
- **LUNDY JS**. Balanced anaesthesia. *Minn Med*, 1926, vol. 9, 399
- **P. H. TONNER**. Balanced anaesthesia today. *Best Practice & Research Clinical Anaesthesiology*, 2005, vol. 19 (3), 475-484
- **WILLIAM R GRECO et al.** The Search for Synergy: A Critical review from a Response Surface Prospective. *PHARMACOLOGICAL REVIEWS*, 1995, vol. 47 (2), 331-385
- **JAAP VUYK et al.** Propofol Anaesthesia and Rational Opioid Selection: Determination of Optimal EC50-EC95 Propofol-Opioid Concentrations that Assure Adequate Anaesthesia and a Rapid Return of Consciousness. *Anaesthesiology*, 1997, vol. 87 (12), 1549-1562
- **AN F. A. HENDRICKX et al.** Is Synergy the Rule? A Review of Anaesthetic Interactions Producing Hypnosis and Immobility. *Anaesthesia & Analgesia*, August 2008, vol. 107 (2), 494-506
- **GURMAN GM**. Assessment of depth of general anaesthesia. Observations on processed EEG and spectral edge frequency. *Int J Clin Monit Comput*, 1994, vol. 11, 185-9
- **DANIEL I. SESSLER et al.** Hospital Stay and Mortality Are Increased in Patients Having a "Triple Low" of Low Blood Pressure, Low Bispectral Index, and Low Minimum Alveolar Concentration of Volatile Anaesthesia. *Anaesthesiology*, 2012, vol. 116, 1195-203
- **GAN TJ** ; **GLASS PS** ; **WINDSOR A** ; **PAYNE F** ; **ROSOW C** ; **SEBEL P** ; **MANBERG P**. Bispectral index monitoring allows faster emergence and improved recovery from propofol, alfentanil, and nitrous oxide anaesthesia. *Anaesthesiology*, 1997, vol. 87, 808-15